# EUROPEAN PATENT APPLICATION

(11) **EP 3 228 629 A1**
(43) Date of publication of application: **11.10.2017**
(21) Application number: 17171175.7
(22) Date of filing: 16.06.2011
(51) Int. Cl.: C07K 14/475, C12N 15/00, C12N 15/62, A61K 38/18, G01N 33/574

(54) **REPROGRAMMING CANCER CELLS**

(30) Priority: 16.06.2010 US 355483 P; 23.09.2010 US 385653 P; 30.12.2010 US 201061428360 P; 17.03.2011 US 201161453917 P; 06.04.2011 US 201161472516 P; 11.04.2011 US 201161474236 P; 09.05.2011 US 201161484052 P
(62) Divisional of application: 11796473.4
(71) Applicant: Minerva Biotechnologies Corporation, Waltham, MA 02451 (US)
(72) Inventor: Bamdad, Cynthia, Boston, MA 02115 (US)
(74) Representative: McNamara, Kathryn

(57) **Abstract**

The present describes a method of treating a patient with cancer or at risk of developing cancer with at least regulatory element that induces differentiation of cells.

SEQUENCE LISTING

<110>  MINERVA BIOTECHNOLOGIES CORPORATION
       BAMDAD, Cynthia

<120>  REPROGRAMMING CANCER CELLS

<130>  13150-70101USA

<160>  6

<170>  PatentIn version 3.5

<210>  1
<211>  45
<212>  PRT
<213>  Artificial Sequence

<220>
<223>  PSMGFR sequence

<400>  1

Gly Thr Ile Asn Val His Asp Val Glu Thr Gln Phe Asn Gln Tyr Lys
1               5                   10                  15

Thr Glu Ala Ala Ser Arg Tyr Asn Leu Thr Ile Ser Asp Val Ser Val
            20                  25                  30

Ser Asp Val Pro Phe Pro Phe Ser Ala Gln Ser Gly Ala
        35                  40                  45

<210>  2
<211>  34
<212>  DNA
<213>  Artificial Sequence

<220>
<223>  primer

<400>  2
atcgatggat ccgatggcca actgtgagcg tacc                                  34

<210>  3
<211>  33
<212>  DNA
<213>  Artificial Sequence

<220>
<223>  primer

<400>  3
gtggtgctcg agttcataga tccagttctg agc                                   33

<210>  4
<211>  30
<212>  DNA
<213>  Artificial Sequence

<220>
<223>  primer

<400>  4
gcaggaacat tatacatggc ggtgattctg                                        30


<210>  5
<211>  30
<212>  DNA
<213>  Artificial Sequence

<220>
<223>  primer

<400>  5
gccatgtata atgttcctgc caacttgtat                                        30


<210>  6
<211>  35
<212>  PRT
<213>  Artificial Sequence

<220>
<223>  Truncated PSMGFR Sequence

<400>  6

Gly Thr Ile Asn Val His Asp Val Glu Thr Gln Phe Asn Gln Tyr Lys
1               5                   10                  15


Thr Glu Ala Ala Ser Arg Tyr Asn Leu Thr Ile Ser Asp Val Ser Val
            20                  25                  30


Ser Asp Val
        35

## Description

### CROSS-REFERENCE TO RELATED APPLICATIONS

The present application claims the benefit of priority to U.S. Provisional Application No. 61/355,483, filed June 16, 2010, U.S. Provisional Application No. 61/385,653, filed September 23, 2010, U.S. Provisional Application No. 61/428,360, filed December 30, 2010, U.S. Provisional Application No. 61/453,917, filed March 17, 2011, U.S. Provisional Application No. 61/472,516, filed April 6, 2011, U.S. Provisional Application No. 61/474,236, filed April 11, 2011, and U.S. Provisional Application No. 61/484,052, filed May 9, 2011, the contents of which are incorporated by reference herein in their entirety.

### BACKGROUND OF THE INVENTION

### 1. Field of the Invention:

The present application describes methods for reprogramming cancer cells thereby treating or preventing cancer.

### 2. General Background and State of the Art:

### Cancer cells are like stem cells

MUC1 is a transmembrane protein that is found on all adult epithelial cells. However, its expression pattern is different on cancer cells than it is on healthy cells. On cancer cells the protein is uniformly spread over the entire cell surface, while on healthy cells its expression is restricted to the apical border. We recently discovered that a transmembrane cleavage product of MUC1, that we named MUC1* (referred to in some previous patent applications as the MGFR) functions as a Class I growth factor receptor on cancer cells¹. The mechanism of action of MUC1* has been elucidated; ligand-induced dimerization of its truncated extracellular domain, comprised essentially of the Primary Sequence PSMGFR sequence (GTINVHDVETQFNQYKTEAASRYNLTISDVSVSDVPFPFSAQSGA (SEQ ID NO:1)) activates growth and survival pathways. MUC1 cleavage releases the bulk of the extracellular domain and a self-aggregation domain that masks the ligand binding site.

We recently discovered that on pluripotent stem cells, virtually all the MUC1 is cleaved to the growth factor receptor form, MUC1*, and that it functions as a growth factor receptor that drives the growth of stem cells². On pluripotent stem cells, the growth factor receptor function of MUC1* is required for their survival. Complete blocking of dimerization of the extracellular domain of MUC1* on stem cells is lethal. However, when stem cells initiate the differentiation process, cleavage of MUC1 is greatly reduced. In fact, newly differentiating stem cells essentially express only full-length MUC1. We showed that the MUC1* form of the protein is a growth factor receptor, whose activation alone is necessary and sufficient for the growth and survival of both embryonic and induced pluripotent stem cells.

On cancer cells, most of the MUC1 is cleaved to MUC1*. As cancer stage progresses, the percentage of MUC1* increases until the cancer cells, like pluripotent stem cells, exclusively present MUC1*. Cancer stem cells express more MUC1* than regular cancer cells³. Cancer cells that have acquired resistance to chemotherapy drugs do so by increasing expression of MUC1* but not of the MUC1-FL (full-length). Pluripotent stem cells secrete MUC1*'s activating NM23 (MNE-7, H1, or H2), which co-localizes with MUC1*. Cancer cells that secrete NM23 and mutants that preferentially form dimers constitutively activate MUC1*-positive cells. Transfection of MUC1* alone is sufficient to transform cells.

### Adult cells can be reprogrammed to revert to pluripotent stem cells

It was recently demonstrated that adult cells could be "reprogrammed" to become pluripotent stem cells. To distinguish these cells from naturally occurring embryonic stem cells, this new type of reprogrammed cell is called an "induced pluripotent cell" or an iPS cell. The first iPS cells were made by transducing cells with four (4) genes. One group inserted the genes for Oct4, Sox2, Klf4 and c-Myc⁴ while the other group inserted genes for Oct4, Sox2, Nanog and Lin28⁵.

All six of these genes, Oct4, Sox2, Klf4, c-Myc, Nanog and Lin28, code for transcription factors, which then regulate the transcription of other genes. The genes that were common to the methods used by both groups were Oct4, Sox2 and by extrapolation Klf4/Nanog because Klf4 is a transcription factor that induces the expression of Nanog. Later work showed that cells could be induced to become pluripotent by inserting only three genes: Oct4, Sox2 and either Klf4 or Nanog. More recent studies have shown that some of these genes could be replaced by adding the proteins that the genes code for rather than the genes themselves. In another study, the genes for Oct4 and Sox2 were transduced and a chemical compound that induced the expression of Nanog were found to induce pluripotency⁶.

### FGF triggers a mouse stem cell pathway and makes human stem cells Primed rather than Naïve (pluripotent)

Recent research has called into question whether or not human stem cells cultured according to state of the art methods are truly pluripotent⁷. In a watershed research article, Jaenisch and colleagues describe human embryonic stem (ES) cells as being "Primed" rather than being true pluripotent stem cells which they term "Naïve". By comparing human embryonic stem (ES) cells to mouse ES cells wherein both were derived from the blastocyst-stage embryos, the researchers discovered that the human ES cells were morphologically and molecularly different from the mouse stem cells. They further disclosed that the human ES cells that have been isolated thus far are not truly pluripotent and more closely resemble mouse stem cells that have been derived from the epiblast stage which is a later stage of development. These findings strongly argue that what we think of as human pluripotent ES cells are not true pluripotent stem cells. Jaenisch and colleagues discovered molecular markers that identify Naive stem cells and markers that identify Primed stem cells.

The researchers were able to temporarily make human Primed stem cells revert to the Naive state by ectopic induction of Oct4, Klf4, and Klf2 factors combined with LIF and inhibitors of glycogen synthase kinase 3β (GSK3β) and mitogen-activated protein kinase (MAP kinase ERK1/2) pathway. Forskolin, a protein kinase A pathway agonist, which can induce Klf4 and Klf2 expression, transiently replaced the need for ectopic transgene expression. Once the human ES cells had been reverted to the Naive state, they needed to be cultured in PD/CH/LIF/FK but could only remain Naive for a few passages before they matured to Primed cells. This is strong evidence that the researchers were not able to identify the growth factors that promote and maintain human ES cells in the pluripotent Naive state.

In contrast to conventional human ES cells these epigenetically converted Naive stem cells gained expression of Klf4, Klf2, Tbx3, Gbx2, Lin28 and SOCS3, hereafter referred to as the Naive markers, and lost expression of Otx2, Sox17, Cer1, Foxa2, Zic1, Lhx2 and XIST, which are markers for the more mature Primed state. In addition, Primed cells that were transiently reverted to the Naive state grew in sheets rather than in colonies. However, this previous research had not been able to identify the growth pathway or the growth factor(s) that made human stem cells propagate as Naive stem cells. Further, even with ectopic expression of genes and growth in a concoction of factors, the reverted cells remained Naive for a short period of time and then progressed to the Primed stage.

Therefore what is needed is a method for propagating human stem cells as Naive stem cells directly after harvest from either an embryo or from an induced pluripotent state, or a method to revert Primed stem cells to the Naive state and then maintain them in that state for prolonged periods of time. What is needed is a method for stably converting Primed stem cells to the Naive state, whereas current methods can only transiently hold the cells in the Naive state. Further, this previously reported method for converting Primed stem cells to the Naive state may not be a complete reversion because they report only observing increases or decreases in the expression of the Naive markers versus the Primed markers. What is needed is a method for converting Primed cells to the Naive state wherein the conversion is complete and they do not express markers of the Primed state. It follows that for efficient directed differentiation of human stem cells, a starting stock of Naive stem cells will be required.

The use of microRNAs for the treatment of cancers holds great promise^{8, 910}. All previous work aimed at identifying regulatory components that differ between pluripotent stem cells, differentiated stem cells and cancer cells have been performed using human stem cells that are Primed and not Naive, rendering the results invalid as a basis for human therapeutics. Until now, researchers claiming to have identified microRNAs that induce differentiation have used stem cells propagated using FGF and mouse feeder cells^{11, 12, 13} which new research shows converts human cells to the Primed state where they are no longer truly pluripotent. Importantly, what is also needed is to perform critical experiments using Naive human stem cells because those performed using human stem cells that were corrupted to the Primed state by FGF and mouse components likely yielded erroneous information not applicable to human medicine.

Until now, cancers have been characterized according to tissue of origin, molecular markers that the cancer cells over-expresses, or growth factor receptors that the cancer cells overexpresses. Yet it is still not enough to predict which patient will respond to a given treatment and, in addition, current treatments are too narrowly focused to completely cure cancer in most cases, with eventual metastasis frequently occurring.

Therefore, there is a need in the art to develop methods to categorize cancers according to their unique signature of regulatory elements that controls their growth and differentiation, so that treatments that reprogram each cancer sub-type can be developed. There is also a need in the art to develop methods for classifying cancer sub-types that allows accurate prediction of groups of patients that will respond to a particular therapy. There is also a need in the art to develop methods for growing and maintaining Naive human stem cells so that the regulatory signatures that control human stem and cancer cell growth and differentiation can be accurately identified and manipulated for anti-cancer treatments.

### SUMMARY OF THE INVENTION

The invention overcomes the above-mentioned problems, and provides methods for growing and maintaining human Naive stem cells, categorizing cancers according to the regulatory networks that control their growth and differentiation and methods for treating cancers or patients at risk of developing cancers by reprogramming or confusing the network that regulates their growth and differentiation.

In one embodiment, cancer is inhibited or prevented by administering to a patient, or one at risk of developing cancer, elements that deliver regulatory signals that induce differentiation. In another embodiment, cancer is inhibited or prevented by administering to a patient, or one at risk of developing cancer, two different elements that deliver two different regulatory signals, wherein one induces pluripotency and the other induces differentiation. In another embodiment, cancer is inhibited or prevented by administering to a patient, or one at risk of developing cancer, a mixture of at least two elements that deliver regulatory signals, wherein those elements are dominant in two cancers of different subtypes. In yet another embodiment, cancer is inhibited by administering to a cancer patient, elements that deliver regulatory signals that are dominant in a different subtype of cancer. For example, a patient with a MUC1*-positive cancer would be treated with elements that deliver regulatory signals that are dominant in a MUC1*-negative cancer. The patient may in addition be treated with elements that deliver regulatory signals that induce differentiation.

In a preferred embodiment, MUC1*-positive cancers are treated or prevented by administering to the patient a microRNA signature of a MUC1*-negative cancer.

In another preferred embodiment, breast cancers and prostate cancers are treated or prevented by administering to the patient miR-145. In one aspect, the invention contemplates treatments that include miR-145 and other microRNAs that are dominant in differentiation, optionally along with microRNAs that are dominant in MUC1*-negative cancers.

Elements that deliver regulatory signals are nucleic acids, RNAs, micro RNAs, genes and gene products. Elements that deliver regulatory signals can be micro RNAs, clusters of micro RNAs, the genes or gene products that they induce, or complementary nucleic acids designed to silence the same set of genes that the micro RNAs silence. In a preferred embodiment, the elements that deliver regulatory signals are micro RNAs and micro RNA clusters.

In another embodiment, signals from more than one cancer type are administered to a patient for the treatment or prevention of cancer.

In one aspect, the present invention is directed to a method of treating a patient suffering from cancer, comprising administering to the patient a composition that contains (i) at least two regulatory elements, or (ii) an agent that causes expression of at least two regulatory elements unique to at least one cancer sub-type signature, wherein the cancer sub-type signature is different from the cancer subtype signature of the cancer that afflicts the patient. The regulatory element may be RNA, microRNA, or protein. The agent that causes expression of at least two regulatory elements unique to at least one cancer sub-type signature is a small molecule. The microRNA may be microRNA-145. The cancer the patient may be afflicted with may be a MUC1*-positive cancer and the regulatory elements unique to the at least one cancer sub-type signature may be from MUC1*-negative cancer cells. In one aspect, the cancer cells of the cancer subtype of the regulatory element may not be co-cultured with the patient's cancer cells.

In a further other aspect, the invention is directed to a method of treating a patient suffering from cancer, comprising administering to the patient a composition that contains (i) at least two regulatory elements, or (ii) an agent that causes expression of at least two regulatory elements unique to at least one stem cell sub-type signature. The stem cell sub-type signature may be that of a newly differentiating human stem cell or a differentiated cell. The stem cell may be Naive stem cell. The cancer the patient is afflicted with may be a MUC1*-positive cancer and the elements unique to the at least one stem cell sub-type signature may be from a newly differentiating human stem cell or a differentiated cell. The stem cell may be Naive stem cell. The regulatory element may be RNA, microRNA, or protein. The microRNA may be microRNA-145. The agent that causes expression of at least two regulatory elements unique to at least one stem cell sub-type signature may be a small molecule.

In another aspect, the invention is directed to a method of treating a patient suffering from cancer, comprising administering to the patient a composition that contains (i) at least two regulatory elements, or (ii) an agent that causes expression of at least two regulatory elements unique to at least one proliferation plateau signature. The regulatory element may be RNA, microRNA, or protein. The microRNA may be microRNA-145. The agent that causes expression of at least two regulatory elements unique to at least one proliferation plateau signature may be a small molecule.

In another aspect, the invention is directed to a method for identifying regulatory element useful to treating cancer in person suffering from cancer, which includes:
(i) obtaining a first molecular composition of regulatory elements from human Naive pluripotent stem cells;
(ii) obtaining a second molecular composition of regulatory elements from cells that have differentiated from the Naive pluripotent stem cells of step (i);
(iii) comparing molecular composition of regulatory elements between the first and second molecular composition of regulatory elements; and
(iv) identifying the regulatory element whose expression is significantly increased in the second molecular composition of regulatory elements.
(v) obtaining a third molecular composition of regulatory elements from the cancer sub-type the patient is afflicted with;
(vi) comparing the regulatory elements identified in (iv) with the third molecular composition of regulatory elements;
(vii) identifying regulatory element that is absent or significantly reduced in the patient's cancer sub-type.

In this method, the regulatory element may be RNA, microRNA or protein.

In another aspect, the invention is directed to a method for identifying regulatory element useful to treating cancer in person suffering from cancer comprising:
(i) obtaining a first molecular composition of regulatory elements from cancer cells;
(ii) obtaining a second molecular composition of regulatory elements from human newly differentiating stem cells;
(iii) comparing molecular composition of regulatory elements between the first and second molecular composition elements; and
(iv) identifying the regulatory element whose expression is increased in the second molecular composition of regulatory elements.

In this method, the first molecular composition may be from the patient's cancer sub-type. The regulatory element may be RNA, microRNA or protein.

In another aspect, the present invention is directed to a method of treating a patient suffering from cancer comprising administering to the patient a regulatory element that induces the growth of a different sub-type of cancer. The regulatory element may be absent from or may be expressed at lower levels in the cancer afflicting the patient compared with the different sub-type of cancer. The regulatory element may be microRNA. The regulatory element may be microRNA-145.

In yet another aspect, the invention is directed to a method for treating a patient with cancer or at risk of developing cancer comprising administering to the patient regulatory elements that are from at least two different and distinct cancer sub-type signatures. The regulatory elements may be chosen from microRNAs, nucleic acids, proteins or small molecules that mimic the activity of the regulatory elements.

In another aspect, the invention is directed to a method of treating a patient with cancer or at risk of developing cancer, comprising administering to the patient at least two separate microRNAs, wherein one induces differentiation and the other induces pluripotency.

In yet another aspect, the invention is directed to a method of treating a patient with cancer or at risk of developing cancer, comprising administering to the patient at least two different microRNAs, or microRNA clusters, wherein each microRNA or microRNA cluster is dominantly expressed in cancers of distinct subtypes. The distinct subtypes of cancer may be MUC1*-positive and MUC1*-negative.

In another aspect, the invention is directed to a method of treating a patient with cancer, comprising administering to the patient microRNA, or microRNA clusters having characteristics of a subtype of cancer that is different from the patient's subtype of cancer.

In still another aspect, the invention is directed to a method for vaccinating a person against cancer by administering to a subject in need thereof a mixture of regulatory elements of at least two cancer sub-type signatures, wherein each uniquely identifies a different subtype of cancer. The regulatory elements may be microRNAs.

In another aspect, the invention is directed to a method for propagating pluripotent stem cells without the need for Rho kinase inhibitor, comprising: culturing the cells with an agent that activates MUC1* growth factor receptor pathway. The agent may be NM23 and may primarily exist in dimer form. This method may further include growing the pluripotent stem cells over human fibroblast feeder layer. This method optionally may further include growing the pluripotent stem cells on a layer free of mouse embryonic fibroblast feeder cell components. Optionally, this method may include culturing the cells on a MUC1* antibody surface, wherein optionally the surface may be Vita^{™} cell culture plate.

In another aspect, the invention is directed to a method for propagating or maintaining Naive stem cells, comprising stimulating MUC1* pathway in the absence of mouse feeder cells or their extracts. This method may optionally further include culturing the cells on a MUC1* antibody surface, wherein optionally the surface may be Vita^{™} cell culture plate.

In yet another aspect, the invention is directed to a method for separating Naive stem cells from a mixed population of cells, comprising contacting the mixed population of cells to a surface coated with MUC1* antibody, wherein the Naive stem cells bind to the MUC1* antibody surface, wherein optionally the MUC1* antibody surface may be formed on a Vita^{™} plate.

In another aspect, the invention is directed to a method of categorizing types of cancers comprising determining which cancer cell types can grow in the conditioned media of another cancer cell type, wherein the ability to grow in the conditioned media of another cancer cell type means that the two cancers belong to the same sub-type.

In still another aspect, the invention is directed to a method of identifying regulatory elements that control the growth and differentiation of a cancer subtype comprising: first categorizing types of cancers according to the methods above, then performing molecular analysis including microRNA analysis and total transcriptome analysis to identify regulatory elements.

In another aspect, the invention is directed to a method of determining suitability of a particular regulatory element treatment for a sub-type of cancer in a patient comprising employing the method according to the methods above to identify the treatment that is effective for that cancer sub-type using the obtained regulatory element information.

These and other objects of the invention will be more fully understood from the following description of the invention, the referenced drawings attached hereto and the claims appended hereto.

### BRIEF DESCRIPTION OF THE DRAWINGS

The present invention will become more fully understood from the detailed description given herein below, and the accompanying drawings which are given by way of illustration only, and thus are not limitative of the present invention, and wherein;

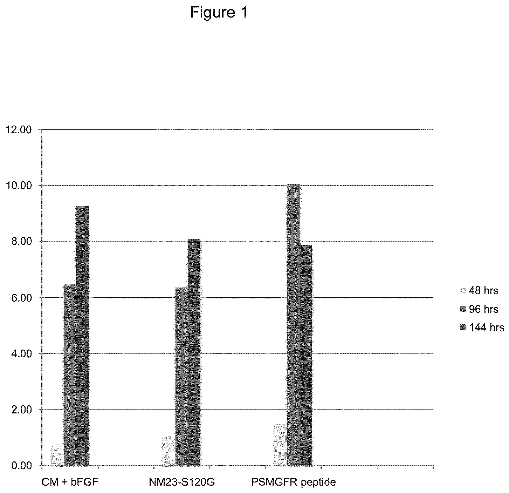

FIGURE 1 shows a graph of the amount of microRNA-145 that is expressed in H9 human stem cells at 48, 96 or 144 hours after cells are allowed to differentiate by withdrawal of bFGF and conditioned media (CM), or NM23-S120G, or when the NM23-MUC1* interaction is purposely disrupted by adding the PSMGFR peptide that is the extra cellular domain of the MUC1* receptor.

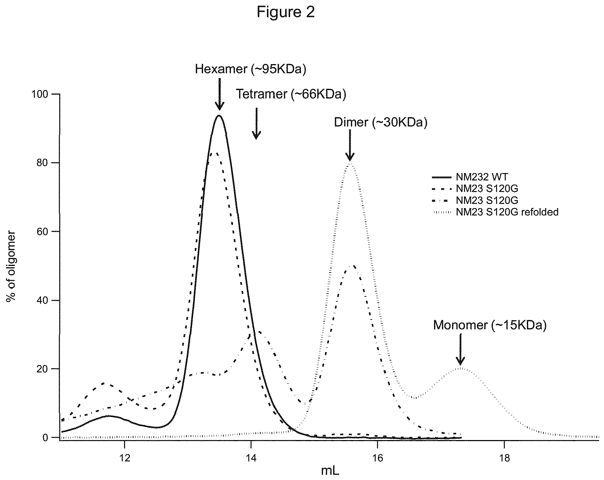

Figure 2 shows an overlay of FPLC traces that show the multimerization state of NM23-WT (wild type), two different batches of NM23-S120G and one batch of denatured then refolded NM23-S120G that resulted in 80% dimer form.

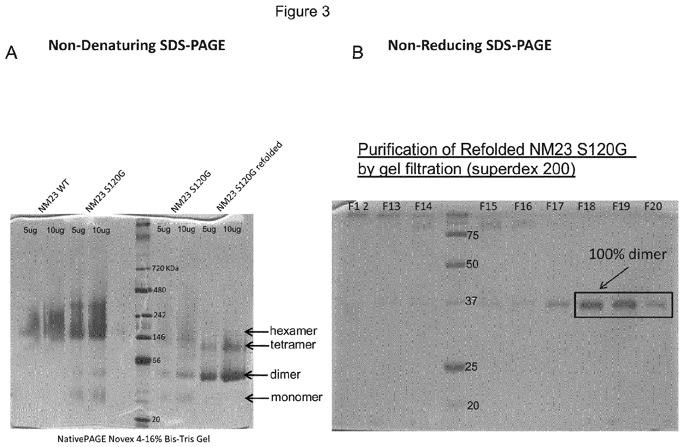

Figure 3A shows a photo of a non-denaturing, native gel showing the multimerization state of different batches of NM23-WT or NM23-S120G.

Figure 3B shows a photo of a non-reducing gel showing that refolded then gel purified NM23-S120G is essentially 100% dimer.

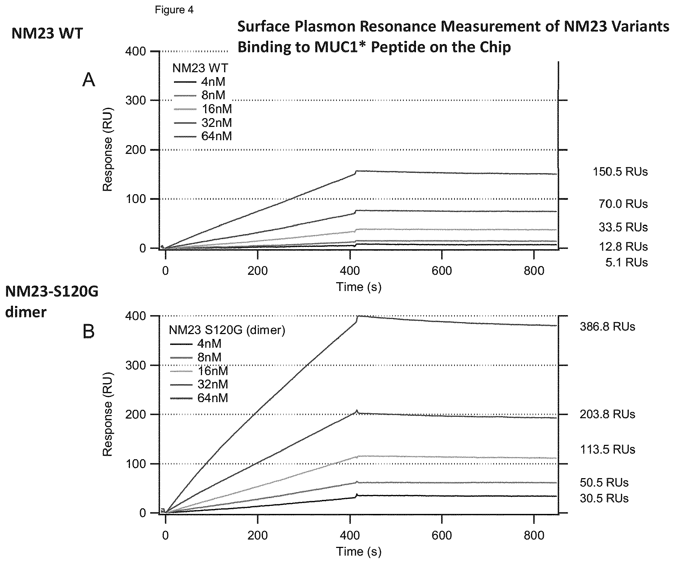

Figure 4 shows sensograms of Surface Plasmon resonance experiments in which either NM23-WT (**A**) or NM23-S120G (**B**) is flowed over an NTA-SAM-coated chip to which the histidine tagged PSMGFR peptide (MUC1* extra cellular domain) was immobilized to saturation. Results show that the NM23-WT which exists as hexamers does not bind to the MUC1* peptide, but the NM23-S120G dimer form does.

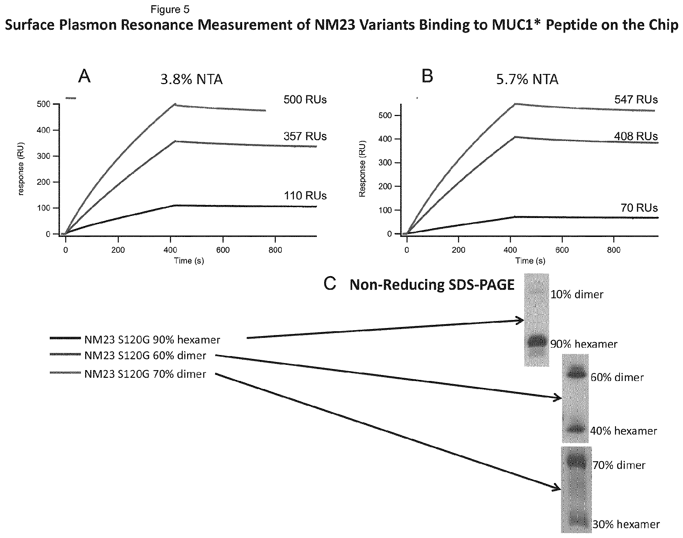

Figure 5 shows sensograms of Surface Plasmon resonance experiments in which different preparations of NM23-S120G are flowed over 3.8% (**A**) or 5.7% (**B**) NTA-SAM-coated chips to which the histidine tagged PSMGFR peptide (MUC1* extra cellular domain) was immobilized to saturation. In part **C**, non-reducing gels show the amount of dimer present in each preparation. Results show that the amount of binding to the MUC1* peptide is a direct function of the amount of dimer present in the sample.

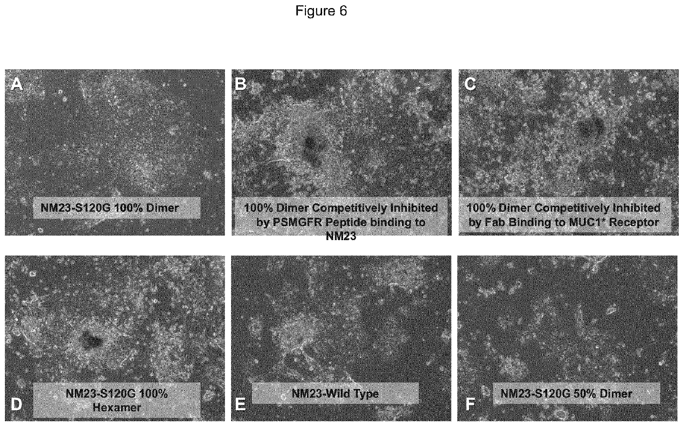

Figure 6 shows 10X photos of H9 human stem cells Day 3 post-plating wherein they have been cultured in different preparations of NM23-S120G characterized by different multimerization states (A-C, D,F), NM23-WT (E), or a NM23-S120G prep that is 100% dimer (A), but competitively inhibited by either the PSMGFR (MUC1* extra cellular domain) peptide (B), or an anti-MUC1* Fab (C). Results show that the hexamer form of NM23, present in NM23-WT, induces differentiation and that interrupting the NM23-MUC1* receptor interaction induces differentiation.

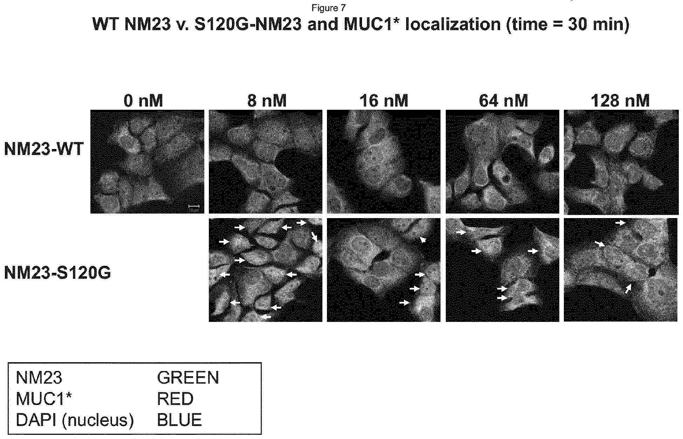

Figure 7 shows confocal microscope images of T47D human breast cancer cells to which has been added either NM23-WT or NM23-S120G mutant comprised of 50% dimers. The results show that the addition of NM23-WT (top row) does not result in increased co-localization with MUC1* receptor and does not increase translocation to the nucleus. Recall that endogenous NM23 is present so a dose-dependent effect is being observed. Conversely, the addition of NM23-S120G (bottom row) co-localized with the MUC1* receptor and is translocated to the nucleus after 30 minutes and the effect is dose-dependent.

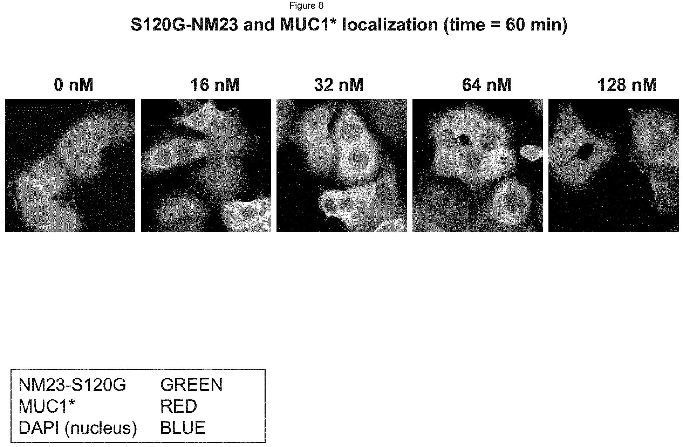

Figure 8 shows confocal microscope images of T47D human breast cancer cells to which has been added NM23-S120G mutant comprised of 50% dimers. Images were taken 60 minutes after introduction of NM23-S120G and show that NM23 and MUC1* receptor co-localize and the effect is dose-dependent.

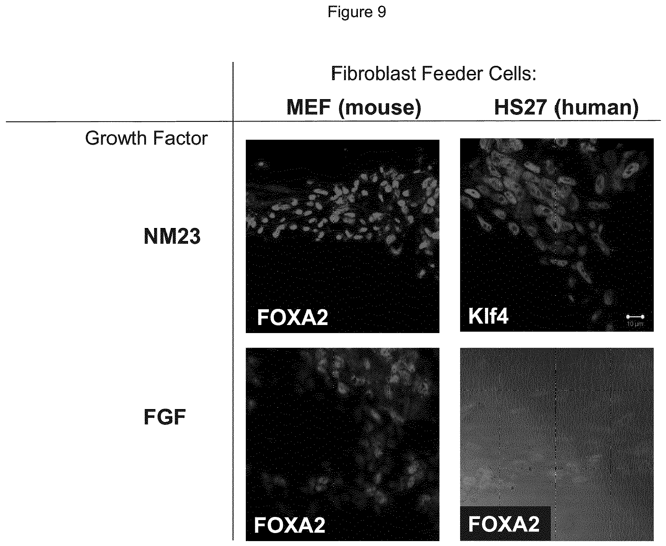

Figure 9 shows confocal microscope images of human H9 embryonic stem cells that have been cultured in NM23-S120G (50% dimer) or bFGF and grown over either human feeder cells or mouse feeder cells, then stained for the presence of the marker of Naive cells, Klf4, and the marker of Primed cells, FoxA2. Results showed that human stem cells cultured in NM23 over human feeders stained positive for K1f4 and negative for FoxA2.

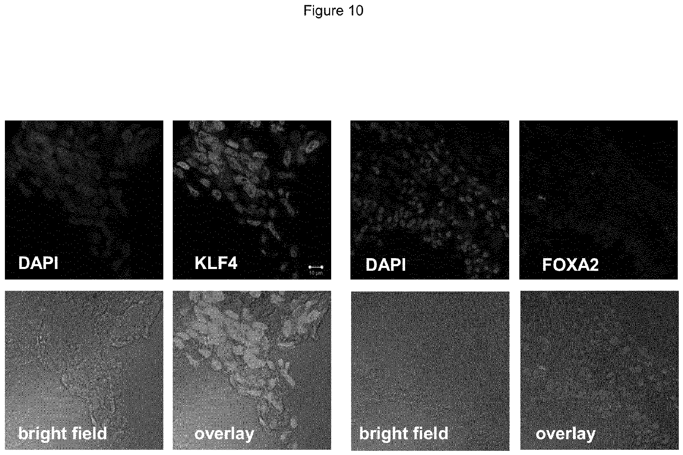

Figure 10 shows confocal microscope images of human H9 embryonic stem cells that have been cultured in NM23-S120G (50% dimer) and grown over human fibroblast feeder cells (HS27). DAPI and bright field images show that virtually every cell in the fields imaged is positive for Naive marker K1f4 and negative for the Primed marker FoxA2.

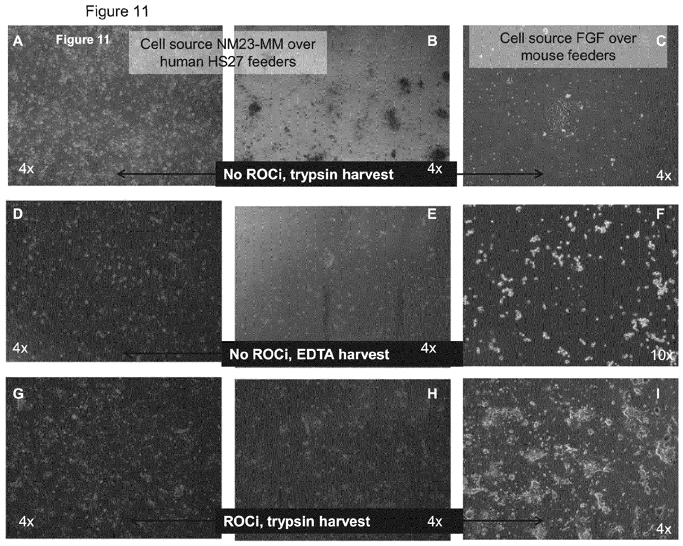

Figures 11A-11I show microscope images of human H9 stem cells (A-E, G-I; 4X, F; 10X) that have been treated with the Rho kinase inhibitor (ROCi Y-27632) (bottom row; G, H, I) or not treated with ROCi. Vita cell culture plates plus anti-MUC1* antibody plates on the left (A, D, G) and right (C, F, I) were centrifuged to bring cells to the surface. Results show that human embryonic stem cells cultured in NM23 over human feeders do not benefit from ROCi and bind plate then grow equally well as ROCi treatment by merely centrifuging briefly to bring cells to the plate surface. Conversely, embryonic stem cells that have been cultured in FGF over mouse feeders do not significantly bind to the plate or grow in the absence of the ROCi.

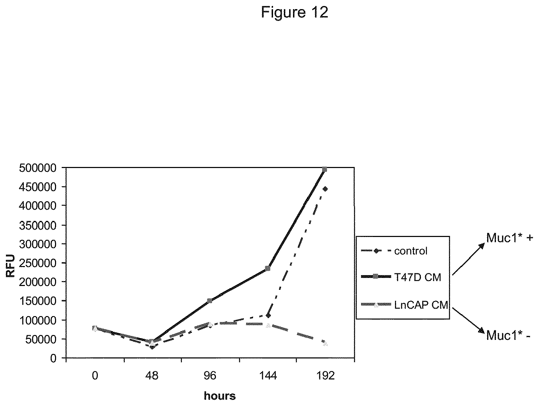

Figure 12 shows a graph of cancer cell growth in response to growth in the presence of conditioned media (CM) from another cancer cell type. The graph shows that one indicator of cancer cell type is if they are MUC1* positive or negative and that MUC1* positive cancer cells do not grow in the presence of conditioned media from MUC1*-negative cancer cells. U87 a MUC1*-positive glioma grows in the conditioned media from MUC1*-positive breast cancer cells (T47D) but not in the conditioned media from MUC1*-negative prostate cancer cells (LnCAP).

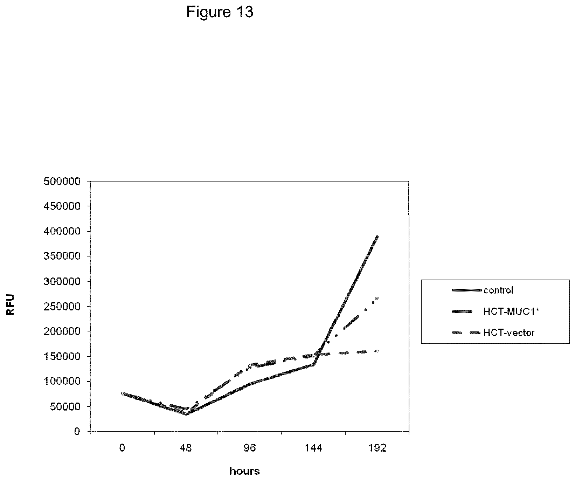

Figure 13 shows a graph of cancer cell growth in response to growth in the presence of conditioned media from another cancer cell type that has been transfected with the MUC1* receptor. U87 a MUC1*-positive glioma grows in the conditioned media from MUC1*-negative colon cancer cells HCT-116 if they have been transfected with gene encoding MUC1* (FLR) but not if transfected with the empty vector.

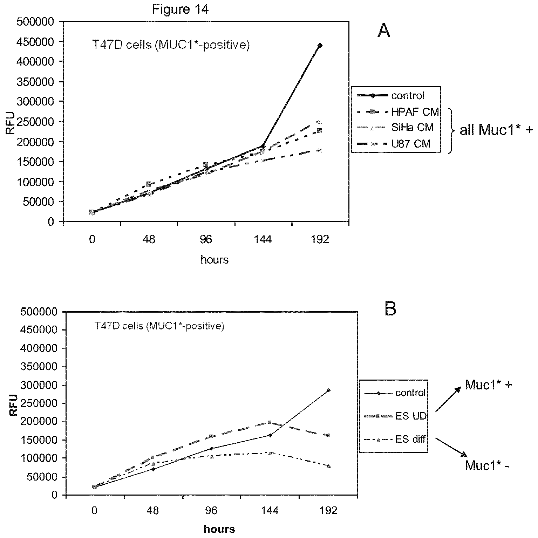

Figure 14 shows a graph of T47D human breast cancer cells that are MUC 1*-positive in response to growth in the presence of conditioned media from other cancer cell types that are also MUC1*-positive (A). Part B shows the growth of T47D cancer cells in response to growth in the presence of conditioned media from either undifferentiated stem cells or newly differentiating stem cells.

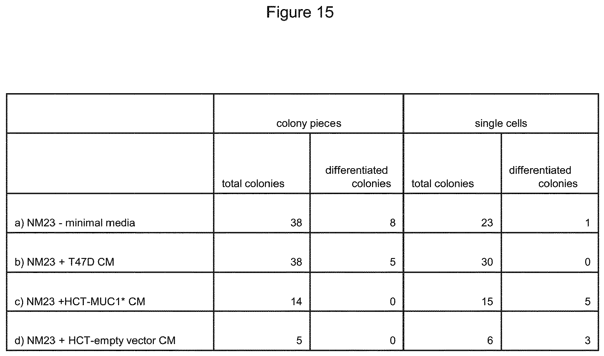

Figure 15 shows a table showing growth of human embryonic stem cells when grown in the conditioned media of MUC1*-positive cancer cells, which improved growth, or MUC1* negative cancer cells plus/minus transfection with MUC1* which both sent the stem cells into stasis. Embryonic stem cells proliferate in the conditioned media from MUC1*-positive cancer cells (T47D and HCT-MUC1*) but not in the conditioned media from MUC1*-negative cells (HCT).

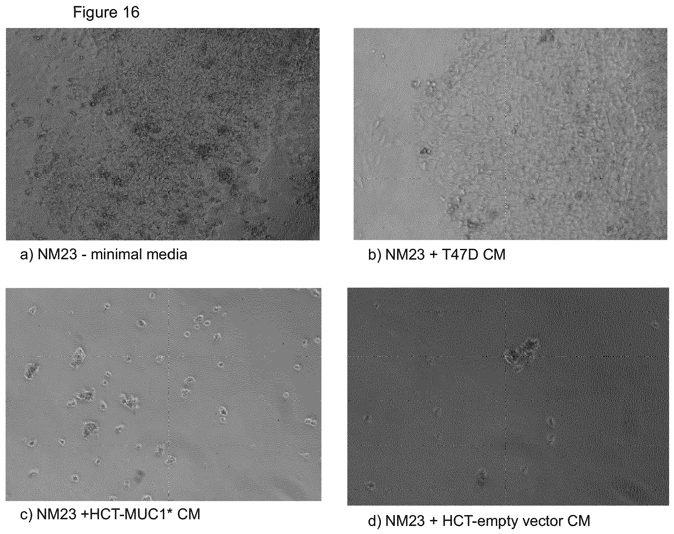

Figure 16 shows photos of human stem cells grown as described in Table of Figure 15 and showing that growth in the conditioned media of MUC1*-negative cancer cells or those that have been transfected with MUC1* send stem cells into stasis.

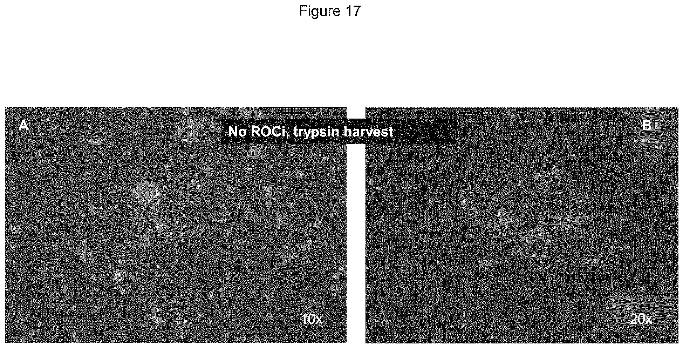

Figure 17 shows photos of human iPS cells grown in NM23-S120 (50% dimer) and plated over Vita plates that had been coated with 12.5ug/ml C3 mab anti-MUC1* antibody as described in Example 7. No Rho kinase inhibitor was added and the plates were not centrifuged to improve cell adhesion yet the iPS cells adhered, proliferated and grew for multiple passages. 17A shows 10x magnification. 17B shows 20x magnification.

### DETAILED DESCRIPTION OF THE PREFERRED EMBODIMENTS

In the present application, "a" and "an" are used to refer to both single and a plurality of objects.

### Definitions

A MUC1-positive cancer cell, also called a MUC1*-positive cancer cell, is a cancer cell that is characterized by the aberrant expression of the MUC1 transmembrane protein which in the healthy state (as well as on MUC1-negative cancer cells) is present only on epithelial cells where its expression is clustered and restricted to the apical border of tissues. Aberrant MUC1 expression, found on MUC1-positive cancer cells, also called MUC1*-positive cancer cells, as used herein, means overexpression and uniform distribution of the protein over the entire tissue surface rather than the clustered pattern of expression at the apical border of tissues, wherein much of the protein is in a truncated form lacking the tandem repeat units.

As used herein, "activating" MUC1* refers to ligand binding to MUC1*, resulting in a stimulation of growth and/or an increase in cell survival.

As used herein, "conditioned media" refers to media in which cells have been grown for a period of time sufficient for a quantity of material to be secreted from the cells into the media..

As used herein, "newly differentiating" stem cells refers to cells transitioning from pluripotency to a more differentiated state.

As used herein, "MUC1*" refers to a membrane-bound MUC1 cleavage product, which is the predominant form of the protein on cultured cancer cells and on cancerous tissues. MUC1* includes the cytoplasmic tail, transmembrane domain, and about 45 amino acids of the extracellular domain (ECD). The exact site(s) of cleavage remain somewhat uncertain.

A primary sequence of this extracellular domain of MUC1* may be composed of but not limited to the amino acid sequence GTINVHDVETQFNQYKTEAASRYNLTISDVSVSDVPFPFSAQSGA (SEQ ID NO:1) (also known as PSMGFR peptide). The term "Primary Sequence of the MUC1 Growth Factor Receptor" (PSMGFR) is a peptide sequence that defines most or all of the MGFR in some cases, and functional variants and fragments of the peptide sequence, as defined below. The PSMGFR is defined as SEQ ID NO:1, and all functional variants and fragments thereof that binds NM23 or an antibody to SEQ ID NO:1, and may have integer value of amino acid substitutions up to 20 (i.e. 1, 2, 3, 4, 5, 6, 7, 8, 9, 10, 11, 12, 13, 14, 15, 16, 17, 18, 19, or 20) and/or any integer value of amino acid additions or deletions up to 20 at its N-terminus and/or C-terminus. As used herein, the abbreviation PSMGFR should not be confused with an amino acid sequence, unless specifically specified.

As used herein, "MUC1 positive cell" refers to a cell that aberrantly expresses MUC1.

As used herein, "MUC1 negative cell" refers to a cell that does not aberrantly express MUC1, including cells that express MUC1 normally.

As used herein, "MUC1* cell" or "MUC1* positive cell" refers to a cell that expresses the truncated form of MUC1, MUC1*.

As used herein, "MUC1* negative cell" refers to a cell that does not express MUC1*, which may include a cell that is MUC1 positive or MUC1 negative, so long as the cell does not express MUC1*.

In some previous patent applications, the transmembrane MUC1 cleavage product has been referred to as the MGFR and as MUC1*. MUC1* and MGFR (MUC1 growth factor receptor) are include the MUC1 cytoplasmic domain, the transmembrane domain and a truncated extracellular domain consisting essentially of the PSMGFR. The contents of US Application Publication No. 2006/0173171, filed August 24, 2004, describing MUC1 activity, and in particular the amino acid sequence of MUC1 and its various fragments is incorporated by reference herein in their entirety.

"Cancer sub-type" as referred to herein is a classification of cancers separated into groups characterized by a unique set of regulatory elements that control growth and differentiation. By unique, it is meant that there are significant differences in expression levels of the regulatory elements among the different cancer sub-types. Unique regulatory elements can be those that have significantly increased or decreased expression levels from one cancer sub-type to another. A collection of unique regulatory elements that are specific to a cancer sub-type are referred to herein as a "cancer sub-type signature".

Subtype signatures are not restricted to cancer cells. Stem cells can be classified into sub-types by similar means. Stem cells and progenitors are analyzed to identify regulatory elements that are unique to each sub-type. By unique, it is meant that there are significant differences in expression levels of that element. Unique regulatory elements can be those that have significantly increased or decreased expression levels from one stem cell sub-type to another. A collection of unique regulatory elements that are specific to a one stem cell sub-type are referred to herein as a "stem cell sub-type signature". Stem cell sub-type signatures can be administered to a person for the treatment or prevention of cancers. Especially preferred are stem cell sub-type signatures wherein they are comprised of regulatory elements that are down-regulated in the cancer that is to be treated or prevented.

As used herein, stem cell "proliferation plateau" refers to growth characterized by expansion of a population of a sub-type of stem or progenitor cells, wherein differentiation to a more mature state is temporarily halted.

As used herein, "proliferation plateau sub-type signature" refers to the collection of regulatory elements that is unique to a sub-type of stem or progenitor cells in a proliferation plateau.

"Regulatory element" as referred to herein can be a nucleic acid, RNA, microRNA, gene, or gene product.

As used herein, "small molecule" refers to a chemical compound preferably having a molecular weight of less than 1KDa, or preferably less than 800Da, and so forth so long as it can be used as an agent to induce expression of a regulatory element or mimic the activity of the regulatory element. Synthesis of such chemical compounds is available to those in the art given the knowledge of the type of assay to use as disclosed in the present application.

As used herein, "significant increase" in regulatory element expression is a term that is known in the art to those in the art of cancer reprogramming.

### Cancer cells and pluripotent stem cells are similar

Growth and survival of both are mediated by the MUC1* growth factor receptor pathway¹. The genes that induce adult cells to revert to become pluripotent stem cells (induced pluripotent stem cells: iPS cells) are genes that regulate transcription of MUC1, its cleavage enzymes and its activating ligand, NM23¹⁴.

MUC1 is cleaved to the growth factor receptor form, MUC1*, on pluripotent stem cells and on cancer cells, where it functions on both as a powerful growth factor receptor. On pluripotent stem cells, either embryonic stem (ES) or induced pluripotent stem (iPS) cells, essentially all of the MUC1 is clipped to the truncated growth factor receptor form, MUC1*. Ligand-induced activation of MUC1* is both necessary and sufficient for pluripotent stem cell growth. NM23 in the dimeric form binds to and dimerizes the extra cellular domain of the MUC1* growth factor receptor, which activates the receptor's growth and survival functions. The addition of nanomolar amounts of NM23, in the dimeric form, on its own fully supports human pluripotent stem cell growth and inhibits differentiation. It is the only known single agent that can support human stem cell growth in the absence of fibroblast feeder cells or their extracts. Once stem cells initiate differentiation, MUC1 cleavage is greatly reduced and little if any MUC1* is detected. Micro RNA-145 (miR-145) signals the transition of stem cells from pluripotency to a more differentiated state¹⁵. Here, we show that specific inhibition of the MUC1*-NM23 interaction causes a profound increase in the expression of miR-145 and a concomitant initiation of differentiation (Fig. 1).

Like pluripotent stem cells, 75% of all solid tumor cancers express MUC1* and their growth and survival is mediated by MUC1*. The addition of the ligand that supports stem cell growth, NM23, also causes cancer cells to grow faster and increases survival. Cancer cells occupy the space between differentiated adult cells and pluripotent stem cells. Cancer cells possess a large degree of "stem-ness". They have self-replicating abilities and have lost many of the characteristics that identify them as particular cell types.

Unlike pluripotent stem cells, in addition to MUC1*, cancer cells can also express full-length MUC1 (MUC1-FL). Importantly, drug resistant cancer cells, which are those that can metastasize, and are also called "cancer stem cells", express more MUC1* than regular cancer cells. When a particular cancer cell acquires drug resistance, it increases the amount of MUC1* that it expresses by 5- to 10-fold. This finding is consistent with the idea that cancer cells are on their way back to becoming stem cells, with the metastatic, highly MUC1*-positive cancer cells being the closest to pluripotent stem cells.

Another similarity between cancer cells and stem cells lies in the identity of the genes that control pluripotency. The pluripotent genes that have been identified that enable the reprogramming of somatic cells back to iPS cells, Oct4, Sox2, and Klf4/Nanog are transcription factors that control the expression of other genes and the proteins they code for. Young and colleagues¹⁶ identified the promoter sites that OCT4, SOX2 and NANOG bind to and identified the genes that were expressed from these promoters. In other words, the genes that are up-regulated by these "pluripotent genes" have been identified. Each transcription factor regulates the transcription of about a dozen genes. Among these were several that the inventor has identified as being MUC1-associated factors. OCT4 and SOX2 bind to the MUC1 promoter itself. SOX2 and NANOG bind to the NM23 (NME7) promoter. NM23-H1 and H2 were previously identified as ligands of MUC1*. Antibodies used in these experiments also recognize NM23/NME7. Therefore, NME7 is an activating ligand of MUC1*. OCT4 and SOX2 both bind to the promoter for MMP16 which we disclose herein is a cleavage enzyme of MUC1. OCT4, SOX2 or NANOG also bind to promoter sites for cleavage enzymes MMP2, MMP9, MMP10, ADAM TSL-1, ADAM TS-4, ADAM-17 (a MUC1 cleavage enzyme), ADAM-TS16, ADAM-19 and ADAM-28, which may also be involved in the cleavage of MUC1. In summary, the genes that induce pluripotency, induce the expression of MUC1, its cleavage enzymes and its activating ligand.

Taken together, these findings strongly argue that cancer cells are very similar to stem cells and MUC1*-positive cancer cells are most similar to pluripotent stem cells.

### Pluripotent stem cells have an inherent program that directs them to differentiate into adult cells.

Stem cells activate a differentiation program when they exit pluripotency. Although cancer cells and stem cells are very similar, cancer cells have become dysregulated such that they do not exit the stem-like, self-replicating state. So one of the paradoxes is, what are the regulatory elements that stem cells activate to exit self-replication, which cancer cells do not activate? By studying how stem cells turn off self-replication, we can learn how to induce cancer cells to also turn off their self-replication program.

One of the key regulators of this primal differentiation program is miR-145. The transition of pluripotent stem cells to the first differentiation state is marked by a large increase in the expression of miR-145¹⁵. Here, we show that interrupting the NM23-MUC1* interaction on proliferating pluripotent stem cells causes an earlier and more pronounced spike in the expression of miR-145 (Fig. 1) with the stem cells immediately initiating differentiation. The result is the same whether the agent added is a MUC1* antagonist or an NM23 antagonist. These results show that both NM23 and MUC1* are also key regulators of the differentiation program. This raises the question of how stem cells interrupt the NM23-MUC1* interaction to initiate differentiation. We have discovered that NM23 is the key player in a self-regulating feedback loop. NM23 can exist as a monomer, a dimer (activating form), a tetramer or a hexamer, depending on its concentration. However, mutants isolated from cancers, such as the S120G mutant¹⁷, have been shown to exist primarily in the activating dimer form and either resist or are unable to form the higher order multimers. We now show that the tetramers and hexamers do not bind to the MUC1* receptor. Therefore, as stem cells proliferate, the concentration and amount of NM23 that they secrete increases. NM23 levels in the medium around proliferating stem cells quickly reaches concentrations where they primarily exist as hexamers, which do not bind MUC1*. NM23 hexamers and tetramers, thus, mimic the action of an NM23 antagonist, resulting in an immediate spike in miR-145 and initiation of differentiation. By contrast, mutants like NM23-S120G, found in a neuroblastoma, that only form dimers, are constitutively active and thus keep the cancer cells in a stem-like self-replicating state. Similarly, this mutant NM23 keeps human stem cells proliferating in the pluripotent state and prevents their differentiation (Examples 2 and 3 below; Figures 2, 3, 6).

Therefore, cancer growth can be inhibited by interrupting the NM23-MUC1* interaction, inhibiting dimerization of NM23, treating with wild type NM23, treating with NM23 mutants, variants or chimeras that are in or preferentially form tetramers or hexamers or by adding regulatory factors, such as miR-145 and the microRNA cluster co-expressed with miR-145, to induce differentiation.

### Cellular reprogramming: it is known that cellular development is bidirectional and that none of the programming elements are ever lost.

Mature cells can be reprogrammed to go back in time and revert to become pluripotent stem cells; stem cells can be directed to differentiate into mature cells. We have shown that cancer cells are like stem cells except that they have lost regulation of the elements that direct them to differentiate and stop self-replicating. It therefore follows that cancer cells can be reprogrammed to differentiate into mature cells that do not self-replicate.

Therefore, it would be a treatment for cancer or for its prevention, to treat with agents that induce cells to differentiate. This can be accomplished by introducing genes, gene products, regulatory nucleic acids, microRNAs or small molecules that induce differentiation or block the effectors of self-renewal. Regulatory elements that induce differentiation, and are therefore suitable as anti-cancer treatments or for cancer prevention, can be identified by: 1) comparing the "stem cell sub-type signatures" of human Naive pluripotent stem cells to differentiated or newly differentiating stem cells (from Naive source); 2) identifying significant changes in the expression levels of regulatory elements between the two wherein the unique regulatory elements in the stem cell sub-type signature of the differentiated cells are those that induce differentiation; 3) comparing the set of unique elements from the stem cell sub-type signature of the differentiated cells to the "cancer sub-type signature"; and 4) identifying those elements that are missing or expressed at significantly different levels in the cancer sub-type signature. These are the regulatory elements that induce differentiation and altered in cancers and are therefore suitable for administering to a patient for the treatment or prevention of cancers.

Regulatory elements that induce differentiation such as RNAs, non-coding RNAs and micro RNAs that regulate the epigenetic processes that induce differentiation or that turn off the genes or gene products that induce pluripotency can be introduced to cancer cells/patients to re-program them to a healthy or more differentiated state. Similarly, these regulatory RNAs, non-coding RNAs and micro RNAs that regulate the epigenetic processes that induce differentiation or that turn off the genes or gene products that induce pluripotency can be introduced to a host animal or a person to either treat cancer or prevent its occurrence. In addition, the invention contemplates the use of small molecules that mimic the effects of the genes, gene products, coding and non-coding nucleic acids or micro RNAs that induce differentiation for the treatment of patients diagnosed with or at risk of developing cancer.

Regulatory elements including nucleic acids, micro RNAs, genes and gene products that induce differentiation can be introduced to cancer cells, or a patient with cancer or at risk of developing cancer, to reprogram the cancer cells back to a more differentiated state and inhibit their ability to self-renew.

Micro RNAs that regulate pluripotency in stem cells can be used to reprogram cancer cells to reverse the cancer process and inhibit their ability to self-renew. We have previously shown that blocking MUC1* receptor dimerization in cancer cells inhibits the growth of cancer cells. Since micro RNAs regulate large networks, they will be more effective at reprogramming cells than single genes, or gene products. Clusters of micro RNAs that induce differentiation would also be effective for the treatment or prevention of cancers.

MicroRNAs that could be used to treat cancers include miR-145, and other micros that induce differentiation. microRNAs that induce differentiation from progenitors to later stages of differentiation are tissue specific. In therapeutic applications, tissue specific microRNAs can be co-administered along with early initiators of differentiation to treat or prevent cancers in specific cell types. For example, miR-128 which induces differentiation in tissues of the brain can be used to treat cancers of the brain. It can be used separately or in combination with other non-tissue specific regulators of differentiation such as miR-145.

Genes or their gene products (the proteins they code for) that are dysregulated by a MUC1 cleavage product can be introduced to cancer cells to "reprogram" them back to a more differentiated state.

In one embodiment, proteins whose expression is decreased in pluripotent stem cells and in cancer cells, are administered to a cancer patient or to a patient as a cancer preventative to maintain the patient's cells in a differentiated state or to "re-program" their cancer cells and induce their return to a more differentiated state. In another embodiment, regulatory nucleic acids whose expression is decreased in pluripotent stem cells and in cancer cells, are administered to a cancer patient or to a patient as a cancer preventative. In a preferred embodiment, the nucleic acid is a microRNA, which can be full-length or mature (the shorter sequence that exists after processing). In yet another embodiment, microRNAs whose expression is increased when stem cells differentiate are administered to a cancer patient or to a patient as a cancer preventative.

Alternatively, down-regulating the genes that induce pluripotency or inhibiting the gene products that induce pluripotency, would be an effective way to treat cancer. Sets of genes or gene products have already been identified that induce pluripotency. Once identified, the set of genes or gene products that induce cells to differentiate will be able to re-program cancer cells such that they lose their self-replication ability.

Both NM23 and MUC1* can function as transcription factors (Figs. 7 and 8). Since the addition of NM23 to growing stem cells maintains them in the pluripotent state, and an increase in miR-145 expression signals the stem cells departure from pluripotency, it follows that the NM23 and/or MUC1* suppress miR-145. The peptide of sequence corresponding to the extra cellular domain of MUC1* (PSMGFR) binds to NM23 or a dimer of NM23 and inhibits its interaction with MUC1* receptor and inhibits its cellular internalization where it interacts with other targets in the nucleus. The addition of the PSMGFR peptide to pluripotent stem cells causes a large spike in the expression of miR-145 which turns on the network that regulates differentiation. Therefore, administering the PSMGFR peptide induces differentiation and thus is a treatment for cancer or for the prevention of cancers. The invention contemplates the administration of natural and unnatural PSMGFR peptides, the administration of vectors or viruses that induce expression of the peptide and peptides that contain sequence variations as long as the peptide is able to bind to NM23. The invention also contemplates the administration of small molecules or antibodies (bivalent, monovalent or bispecific, natural or engineered antibodies and antibody fragments) that inhibit the interaction between NM23 and MUC1*. Additionally, we note that we have shown that the NM23 dimer is the form that activates the MUC1* growth factor receptor pathway. NM23 at higher concentrations, forms tetramers and hexamers which we find do not bind to MUC1*. Therefore, we typically use a mutant such as NM23-S120G that inhibits tetramer and hexamer formation for stimulating stem cell growth. However, the wild type NM23 that forms the higher order multimers initiates a feedback loop that turns off pluripotent growth and initiates differentiation. Therefore, the invention also contemplates using wild type NM23 for the treatment and prevention of cancers.

It is then therefore important to be able to identify agents that induce differentiation so that they can be administered as anti-cancer agents. Similarly, it is important to be able to identify agents that induce pluripotency so that they can be inhibited for the treatment or prevention of cancers.

### Regulatory Elements that make up a sub-type signature can be identified as follows.

Identification of naturally occurring agents that regulate growth and differentiation can be accomplished by a variety of techniques known to those skilled in the art. For example, agents that induce differentiation can be identified by virtue of the fact that they will be: a) absent or greatly reduced in pluripotent stem cells; b) present in newly differentiating stem cells; and c) absent or greatly reduced in cancer cells. Once identified, naturally occurring agents that induce differentiation can be mimicked through the use of small molecules, using techniques known to those skilled in the art. The kind of molecular comparisons that are employed to identify agents that induce differentiation will vary. To identify regulatory nucleic acids and microRNAs, one would separately extract RNA from each set of cells (pluripotent, differentiating, or cancer) or their conditioned media and compare using techniques such as Deep Sequencing. To identify genes and gene products that are regulated by an agent that induces differentiation and that is known or suspected of being a transcription factor, one would use assays like ChIP (chromatin immuno precipitation assays) or techniques such as ChIP Seq. Comparison of the proteins and nucleic acids that each cell type secretes can also be done to identify secreted proteins that induce differentiation, (see Example 5 below).

On the other hand, interactions that promote self-replication are purposely activated so that regulatory agents that override differentiation can be identified. These self-replication regulatory agents may be inhibited for the treatment of cancers. Alternatively, they may be used to promote the proliferation of stem or progenitor cells *in vivo* or *in vitro.* As will be described below herein, some regulatory elements that induce self-replication may be used for the treatment of cancers to deliver a "mixed signal".

Alternatively, interactions that induce self-replication are purposely disrupted so that a change in the molecular make up of the cells becomes evident; disruption of the interaction increases production of regulatory agents that inhibit self-replication and therefore can be used for the treatment of cancers or to direct differentiation of stem cells.

In one embodiment, the interaction between NM23 and MUC1* is disrupted, for example using an Fab of an antibody raised against the PSMGFR portion of the MUC1* extra cellular domain or a peptide having the sequence of the PSMGFR. Molecular analyses including Deep Sequencing and transcriptome analyses is performed on cells with and without NM23-MUC1* interaction disrupted. Molecular analyses are performed to identify agents (nucleic acids, microRNAs, or proteins) that are upregulated when interaction is disrupted; agents that are upregulated are agents that induce differentiation.

In addition to the activity of secreted NM23 on MUC1* extra cellular domain, we have discovered that NM23 bound to the MUC1* extra cellular domain is translocated to the nucleus of the cell where they act as transcription factors or co-factors that upregulate genes that induce pluripotency (Example 4 below, and Figs. 7 and 8). ChIP assays and ChIP seq assays are performed to identify pluripotency genes that are induced by NM23-MUC1* or differentiation genes that are suppressed by them. Dimeric NM23 when added to stem cells or cancer cells binds to the extra cellular domain of MUC1*, then both are translocated to the nucleus within 30 minutes. During that nuclear localization period, antibodies that bind to MUC1* and/or NM23 would be added to precipitate out the proteins and nucleic acids to which they are bound. In a preferred embodiment, antibodies directed against the PSMGFR region of MUC1 are used in assays to determine which nucleic acids MUC1* binds to, in for example ChIP assays. Alternatively, antibodies that bind to the MUC1 cytoplasmic tail can be used. After identifying the nucleic acid sequences that are bound by MUC1* one could use any one of a number of standard techniques such as RT-PCR, Deep Sequencing, transcriptome analysis and the like, to determine whether the nucleic acid sites to which MUC1* binds cause the up- or down-regulation of which genes or gene products.

It is clear that dimerization of MUC1* by a bivalent antibody or by its activating ligand, NM23, signals the cell to remain or become stem-like. Our evidence is that either anti-MUC1* antibody or dimeric NM23 drives the growth of cancer cells and pluripotent stem cells and inhibits the differentiation of stem cells. It is also clear that blocking, or suppressing expression of, MUC1* signals the cell to leave pluripotency or differentiate. Therefore, to identify regulatory elements that induce pluripotency, one would stimulate first set of pluripotent stem cells with a MUC1* stimulator and identify micro RNAs, genes or gene products that are upregulated. To highlight which ones are up-regulated in pluripotency, one would compare these results to a second set of stem cells in which MUC1* signaling is blocked, for example, by adding the MUC1* extra cellular domain peptide (PSMGFR), which induces differentiation. RNAs, micro RNAs, genes or gene products that are highly expressed when MUC1* is activated, but expressed in low amounts when MUC1* is blocked, are regulatory elements that induce pluripotency. RNAs, micro RNAs, genes or gene products that are expressed in low amounts when MUC1* is activated, but high when MUC1* is blocked, are regulatory elements that induce differentiation, and such are suitable for use in treating or preventing cancers. This process is performed on MUC1*-positive cancer cells to identify cancer sub-type signatures and also on pluripotent stem cells to identify stem cell sub-type signatures.

MUC1* expression is exemplary and can be used as a model for identifying genes, gene products or microRNAs that can be introduced to reprogram cancer cells so that they become more like differentiated cells. The invention contemplates performing similar analyses with other agents that promote self-replication or differentiation.

Regulatory elements such as microRNAs and their targets that are down-regulated in cancers can be used to modulate the growth and differentiation state of cancer cells as an anti-cancer treatment or as a vaccine for the prevention of cancer. MUC1* expression is high in pluripotent stem cells and in many cancers. In addition, its expression is even higher in aggressive or metastatic cancers, which are more stem-like than less aggressive cancers. Agents that antagonize the interaction between MUC1* and its activating ligand, NM23, induce differentiation. These characteristics make MUC1* and elements that regulate its expression ideal therapeutic targets for reprogramming cancer cells.

Characteristics that will identify other targets that should be inhibited or down-regulated for treatment or prevention of cancers are: 1) high expression in pluripotent stem cells and cancer cells; 2) higher expression in aggressive cancers than in less aggressive cancers; 3) absence in mature, differentiated cells; and 4) demonstration that inhibiting its expression induces differentiation. Thus, to reprogram cancer cells to induce a more differentiated state, one would introduce inhibitors of the expression of MUC1, MUC1*, its activating ligand NM23, or its cleavage enzymes that include MMP-14, MMP-16, and TACE also called ADAM-17. Because microRNAs exert control over large networks of interrelated genes and gene products, microRNAs that regulate expression of the targets, for example, MUC1, its cleavage enzymes and NM23, are preferred as the therapeutic agent to reprogram cancers or prevent their occurrence. Thus, miR-145 is one of the microRNAs that could be used alone or with other micros to treat or prevent cancers.

### Previous attempts to identify microRNA signatures of differentiation were flawed, making them unsuitable as human therapeutics.

All studies attempting to identify microRNAs that direct differentiation for the potential treatment of cancers have been performed using stem cells grown by activating the FGF pathway and also wherein most used mouse fibroblast feeder cells, their conditioned media or Matrigel, which is derived from mouse tumor. Researchers recently showed that activation of the FGF pathway maintains mouse stem cells in the pluripotent state but causes human stem cells to enter a state called "Primed", which is no longer pluripotent and is not representative of naturally occurring human stem cells. Therefore, to accurately identify regulatory agents that induce differentiation of human stem cells for the treatment of human cancers, one must compare truly pluripotent (Naive) human stem cells to newly differentiating human stem cells (wherein source was Naive) and then to human cancer cells, wherein regulatory agents like microRNAs that are absent in Naive stem cells, present in newly differentiating stem cells and absent in human cancers are identified and used as therapeutic agents for the treatment or prevention of cancers.

Researchers have been able to force human Primed stem cells into the true pluripotent, Naive state, for short periods of time, by inserting genes and various chemical agents. Characterization of these temporary Naive human stem cells showed that they grow in sheets rather than colonies, and they can be harvested by trypsinization without negative effects, express higher levels of KLF4 than Primed stem cells and express lower levels of FOXA2. Until now, it has been unknown what the pathway is that maintains growth of human Naive stem cells. Here we present compelling evidence that human stem cells cultured in NM23 and via activation of MUC1* growth factor receptor, in the absence of FGF, mouse fibroblast feeder cells, their conditioned media or Matrigel, are in the Naive state and thus are the true pluripotent human stem cells. Therefore, it is only through the analysis of human Naive stem cells, obtained by culturing according to methods of the invention, and then allowing those cells to differentiate by withholding NM23, that an accurate identification of regulatory agents that induce differentiation of human cells can be made. Although some regulatory elements, such as microRNAs, which were previously identified using FGF-grown or mouse feeder layers derived stem cells, may be correct, it is impossible to distinguish between those that are correct and those that are artifacts of a system using stem cells that are not true pluripotent human stem cells. Further, therapeutic uses of regulatory elements that are altered in cancer and that induce differentiation will be more effective when used in combinations of two or more of such regulatory components or signatures. A therapeutic treatment comprising a signature of regulatory elements in which some will have a positive effect, some a negative effect and some no effect, would not be suitable as a human therapeutic. Therefore, it is critical that real pluripotent human stem cells (Naive) are used to identify regulatory elements that are altered when the cells transition to a more differentiated state, and wherein further comparison to a cancer sub-type signature reveals the identity of those elements that are missing or expressed at incorrect levels in cancers; a therapy in which the expression levels of these elements is restored in cells constitutes an effective therapy for patients with cancers or for the prevention of cancers. The invention contemplates the use of stem cells cultured via MUC1* stimulation for the studies described herein that are used for the treatment of cancers, for culturing human stem cells or for directing their differentiation.

Our experiments show that when we culture human embryonic (ES) or induced pluripotent stem (iPS) cells in NM23 without bFGF or mouse feeder cells or Matrigel, they revert to the Naive state (Examples 6 and 7 below; Figs. 9, 10 and 11). The stem cells stain positive for the markers of the Naive state, Klf4 and negative for the markers for the Primed state, such as FoxA2. In addition, human stem cells cultured in NM23, devoid of bFGF or mouse feeder cells or their conditioned media, plated over a surface of anti-MUC1* antibody surface, grow in sheets rather than in colonies, which is another characteristic of Naive stem cells. We have also shown that the addition of Rho kinase inhibitor or MAP kinase inhibitor accelerates the process of reversion to the Naive state.

We have thus described an approach for treating or preventing cancers wherein agents that induce cellular differentiation are administered to the patient to limit cancerous, self-replicating growth. An alternative approach is to treat or prevent cancers by administering to the patient at least one of a set of first regulatory agents that is characteristic of a first type of cancer and at least one of a set of second regulatory agents that is characteristic of a second type of cancer. That is to say a first cancer sub-type signature and a second sub-type signature.

We discovered that certain subtypes of cancer cells cannot be co-cultured and will not grow in the presence of conditioned media of the other sub-type of cancer. The conditioned media contains secreted factors that can program the cells to grow in a certain way. The fact that certain types of cancer cells cannot grow in the presence of the conditioned media of another specific type is consistent with the idea that the cells are proliferating according to two different programs and that when a cells receives a mixed signal, meaning its own proliferation program plus that of another, the cell stops proliferating and goes into stasis.

For years it has been observed that people do not contract multiple cancer types at the same time; one cancer type metastasizes instead. These data together with our own experiments and observations, leads to the conclusion that treating a cancer patient, or a patient at risk of developing cancer, with fundamentally mixed signals, wherein the signal(s) constitutes a part of regulatory signatures of different proliferation plateaus, will inhibit the cancer cells' ability to proliferate and would be a powerful anti-cancer treatment.

Until now, cancers have been characterized according to tissue of origin, molecular markers that the cancer cells overexpress, or growth factor receptors that the cancer cells overexpress. However, now researchers are looking at which microRNAs various tumors over- or under-express. Here, for the first time, a method for characterizing cancers is described wherein cancers are classified according to which other cancer cells they can be co-cultured with. Cancers that can be co-cultured together or grown in the conditioned media of the other belong to the same sub-type of cancer and conversely cancer cells that cannot be co-cultured belong in different cancer sub-groups. Cancers should first be sub-type classified in this way and representative cancers from each sub-type are then analyzed to identify regulatory elements that are unique to each sub-type. By unique, it is meant that there are significant differences in expression levels of the element. Unique regulatory elements can be those that have significantly increased or decreased expression levels from one cancer sub-type to another. A collection of unique regulatory elements that are specific to a cancer sub-type are referred to herein as a "cancer sub-type signature". A cancer patient can be treated by administering to the patient a cancer sub-type signature of a cancer that is different from the one that afflicts the patient, which is referred to herein as a "foreign cancer sub-type signature". The invention also contemplates treating the patient by administering to the patient more than one foreign cancer sub-type signature. The invention further contemplates administering to a healthy person, for the purpose of preventing cancers, at least two cancer sub-type signatures.

For example, if one identified a microRNA signature of one cancer sub-type and another microRNA signature of another cancer sub-type, where few of the microRNAs are present at comparable levels in both cancer sub-types, then the two clusters of microRNAs would be administered to a patient with cancer or at risk of developing cancer to send the cell a mixed signal which results in the cell going into a static, non-proliferating state. In a similar approach, one would identify at least two different microRNA signatures that induce two different cell types to differentiate into different cell fates, e.g. a first set of micros that induce cells to differentiate into neurons and a second set of micros that induce cells to become skin cells. These would be two different proliferation plateau signatures that would be together administered to a patient to treat or prevent cancer by delivering two regulatory signals that if delivered to an immature, differentiating cell, would cause the cells to go into a static state to prevent the occurrence of mutant species. In another approach, the two conflicting regulatory signals that would be delivered to treat or prevent cancer would be a first set of micros that induce pluripotency and a second set of micros that induce differentiation. These are examples of two different stem cell sub-type signatures that can be used for the treatment or prevention of cancers. In yet another embodiment, the patient is treated with molecules that make up only one stem cell sub-type signature, for example a set of microRNAs which then conflicts with the cancer sub-type signature, for example microRNA signature, of the patient's own cancer cells, which would inhibit their stem-like proliferation.

### Pluripotent stem cell differentiate into progenitors which are precursors of cells that eventually differentiate into terminally differentiated adult cells

We previously reported that MUC1 is clipped to the pluripotency growth factor receptor, MUC1*, in pluripotent stem cells, but reverts to the quiescent full-length MUC1 as soon as the stem cells begin to differentiate. We further discovered that MUC1 cleavage to MUC1* resumes again at some key progenitor stages to expand specific progenitor populations before further differentiation. For example, expression of the pluripotency growth factor receptor MUC1* is suppressed at the onset of differentiation but is expressed again on hematopoietic stem cells, neural stem cells and others, including retinal cells, where there is a need to expand important sub-populations before they differentiate further. The expansion of other later stage progenitor states is controlled by growth factor receptors other than MUC1*.

### Progenitors stop differentiating for a finite period of time and expand their sub-population before further differentiating.

We call these stem-like expansion periods "proliferation plateaus". By stem cell "proliferation plateau" is meant growth characterized by expansion of a population of a sub-type of stem or progenitor cells, wherein differentiation to a more mature state is temporarily halted. There are several proliferation plateaus that expand different sub-populations and each one will be characterized by a set of regulatory elements that direct a self-replicating program. By "proliferation plateau sub-type signature" is meant the collection of regulatory elements that is unique to a sub-type of stem or progenitor cells in a proliferation plateau. MUC1* plays a role in the regulation of growth and differentiation of a few early progenitors, however, others will be characterized by their own unique proliferation plateau sub-type signatures. Just as pluripotent stem cells share much of their stem cell sub-type signature with the cancer sub-type signature of MUC1*-positive cancer cells, other cancer sub-type signatures will have significant overlap with specific proliferation plateau sub-type signatures. To identify regulatory elements that comprise a signature when little is known about its growth or differentiation pathways, techniques such as Deep Sequencing or total transcriptome analysis, and can be used to generate signatures.

Our evidence indicates that cancers occur when mature cells errantly become trapped in a proliferation plateau. Therefore, cancer sub-type signatures can be identified by performing molecular comparisons between the cancer cells and various progenitors. Each cancer sub-type signature will be close to a stem cell sub-type signature or a proliferation plateau sub-type signature. Our experiments show that some sub-types of cancer cells cannot proliferate in the presence of cancer cells of another sub-type. Each particular sub-type of cancer cell secretes agents that turn on the program that mediates growth of the particular proliferation plateau that they are trapped in. When a cancer cell receives signals that turn on its own proliferation plateau, its "proliferation plateau sub-type signature" or its "cancer sub-type signature" plus a foreign proliferation plateau or a foreign cancer sub-type signature, the cell becomes confused and stops proliferating. This means that cancer growth can be inhibited by treating the cells (patient) with a foreign cancer sub-type signature or a proliferation plateau signature that may or may not have elements that are common to both the proliferation plateau signature and the cancer sub-type signature.

MUC1*-positive cancer cells are trapped in the regulatory network that makes pluripotent stem cells proliferate. The cancer cell sub-type signature of MUC1*-positive cancer cells is therefore very similar to the stem cell sub-type signature of pluripotent stem cells. Therefore MUC1*-positive cancer cells secrete regulatory elements that induce pluripotency, while MUC1*-negative cancer cells do not because they secrete factors that expand a different progenitor population. The cancer sub-type signature of a MUC1*-negative cancer cell will be very similar to the stem cell sub-type signature of one of the progenitor cell populations. Both cancer cell types, like both stem cell types, have the ability to self-replicate, but they are controlled by different regulatory elements. We have demonstrated that a layer of MUC1*-positive cancer cells or their conditioned media can fully replace the need for fibroblast feeder cells or their conditioned media or bFGF for the growth of pluripotent stem cells. However, MUC1*-negative cancer cells or their conditioned media cannot support the growth of pluripotent stem cells. This means that mixing elements of the cancer sub-type signature of MUC1*-negative cells interferes with elements of the stem cell sub-type signature of the pluripotent stem cells and the mixed signal results in stem cell stasis or death. Since we have shown that pluripotent stem cells and MUC1*-positive cancer cells can be co-cultured and that either can grow in the conditioned media of the other, it is concluded that their sub-type signatures are very close. Therefore, just as the cancer sub-type signature of MUC1*-negative cells caused stasis and death of pluripotent stem cells, they will cause stasis and death of MUC1*-positive cancer cells. This finding supports the conclusion that cancers are a collection of diseases caused by a population of mature cells getting trapped in a stem or progenitor "proliferation plateau" and cancers can be effectively treated by administering to the patient regulatory elements of a different cancer sub-type signature or of a different stem cell sub-type signature.

In a reciprocal experiment, we observed that MUC1*-positive cancer cells proliferated as usual when cultured in the conditioned media from pluripotent stem cells but went into stasis and did not proliferate when cultured in the conditioned media of newly differentiating stem cells. This finding is consistent with the idea that they did not proliferate because the regulatory factors secreted by the differentiating stem cells reprogrammed the cancer cells so that they differentiated and lost their ability to self-renew.

The finding is also consistent with the idea that nature has developed mechanisms to prevent the occurrence of mutants; differentiating stem cells that receive mixed signals enter a static phase and do not multiply. For example, a stem cell that simultaneously received signals to become a blood cell and another signal directing it to become a muscle cell would trigger a default program to prevent that cell from further dividing. Since cancer cells are stem cells that have lost the programming that tells them to differentiate, those cells could be caused to go into a static state and cease multiplying by sending the cells a "mixed" proliferation plateau signal.

The current state of the art method for culturing pluripotent stem cells is to grow them over a layer of or in the conditioned media of fibroblast "feeder" cells plus fibroblast growth factor (bFGF). If it is correct that cancer cells are stem-like and their growth and differentiation state is being regulated by many of the agents that maintain pluripotency, then we reasoned that stem cells could be grown in the conditioned media of or over a layer of cancer cells. Because MUC1* is a key modulator of the growth and differentiation of stem cells and cancer cells, we further predicted that the conditioned media from MUC1* positive cancer cells would be best for growing and maintaining stem cells in the pluripotent state. Our experiments showed that pluripotent stem cells grow well in the conditioned media from MUC1*-positive cancer cells, but not in the conditioned media from MUC1*-negative cancer cells. This finding confirmed our idea that cancers are a collection of diseases that are basically caused by a population of mature cells being trapped in a progenitor proliferation plateau.

Nature has developed mechanisms to prevent the occurrence of mutants; differentiating stem cells that receive mixed signals enter a static phase and do not multiply. For example, a stem cell that simultaneously received signals to become a blood cell and another signal directing it to become a muscle cell would trigger a default program to prevent that cell from further dividing. Since cancer cells are mature cells that are being reprogrammed to become stem-like, those cells could be caused to go into a static state and cease multiplying by sending the cell a "mixed" signal that conflicts with the signal that the cell is operating under. In support of this hypothesis, researchers recently reported that immunizing animals with pluripotent stem cells prevented their getting a colon cancer. Here, the pluripotency signals given off by the pluripotent stem cells conflicted in a fundamental way with the partially differentiating signals of the cancer cells.

In nature, pluripotent stem cells proliferate, then differentiate on their way to becoming fully mature cells, which are referred to as terminally differentiated cells. Along this pathway from pluripotent to terminally differentiated, it appears that there are proliferation plateaus, wherein at key progenitor stages, there is a need to expand the cell population before entering the next step in differentiation. It is our hypothesis that cancers make up a collection of diseases where the cell's programming becomes confused and they are trapped in one of the several stem-like proliferation plateaus. If this hypothesis were true, and the cancer cells could be forced into a static state by sending simultaneous stem-like proliferation signals, then it follows that the conditioned media from different kinds of cancer cells would contain some of the programming factors and co-culturing of differentiate types of cancer cells would cause the cells to receive mixed signals which would prevent proliferation.

As evidence of the validity of this theory, we discovered that several types of cancer cells cannot grow in the presence of other types of cancer cells. We reasoned that since pluripotent stem cells require signaling through MUC1* receptor to grow, that MUC1* positive cancers would constitute a category of cancers that are far along the pathway toward complete stem-like behavior and that MUC1*-negative cancers would be of a different class. We therefore grew either MUC1*-positive or MUC1*-negative cancer cells in the conditioned media of the opposite type of cancer cell. Figure 12 shows that U87 cells (MUC1*-positive glioma) cannot grow in the presence of conditioned media from LnCAP cells (MUC1*-negative prostate cancer cells). However, U87s grow as they normally would in the conditioned media of MUC1*-positive breast cancer cell line T47D (Example 8 below). The cells were cultured for 192 hours (8 days) with noticeable differences in growth appearing at about 96 hours (4 days), which corresponds well to previously published reports of the time required for micro RNA networks that signal a change to arise¹⁵. There was a greater than 10-fold difference between the growth of the U87 in conditioned media from MUC1*-positive cancer cells and that of MUC1*-negative cancer cells, demonstrating that elements that deliver regulatory signals in one type of cancer, inhibit the growth of a different subtype of cancer. Note that to correct for any effect of different media on cell growth, because each cell type has its own recommended media for growth, all the cells were grown in the same media: 50% of the media is the media recommended for the growth of the cells on the plate and the other 50% is the media that the other cell type was grown in, such as conditioned media.

### Cancers can be treated by administering to the cells/patient agents that initiate differentiation, which stops the self-replicating process and stops cancer cell growth.

Figure 13 shows that U87 cells (a MUC1*-positive glioma) grow in the conditioned media from MUC1*-negative colon cancer cells HCT-116 if they have been transfected with MUC1* (FLR) but not if transfected with the empty vector. To further this point, we cultured U87 cells in either the conditioned media of MUC1*-negative cancer cells, HCT-116 (colon cancer line) or in the conditioned media of the HCT-116 cells that had been transfected with gene encoding MUC1*. Figure 13 shows that the conditioned media of a MUC1*-negative colon cancer cell line, HCT-116 transfected with an empty vector ("HCT-vector") did not allow the growth of the MUC1*-positive U87 cells after about 96 hours, which is the time required to activate a microRNA regulatory network¹⁵. In contrast, the conditioned media from the same cell line, HCT-116, except that it had been transfected with a vector encoding MUC1* ("HCT-MUC1*") did allow the growth of the U87s (Fig. 13). Note that all the cells were grown in the same media which is a 50/50 mix of the media recommended for the cells on the plate and the media used for the cells contributing the conditioned media. The "control" cells are cultured in 50/50 mix of the U87 media and minimal stem cell media.

Figures 14A-14B show that T47D cells (MUC1*-positive) proliferate in the conditioned media of other MUC1*-positive cancer cells (Fig. 14A); and they do not proliferate in conditioned media from newly differentiating stem cells (Fig. 14B). The T47D cells reach stasis after 6 days when cultured in conditioned media from undifferentiated stem cells which are MUC1*-positive, indicating delivery of a mixed cancer and pluripotency signal triggers a static growth state.

### Identification of regulatory agents that are in mature or maturing cells, that have been lost in cancer cells

To identify micro RNAs that dominate pluripotency, one may perform standard techniques such as Deep Sequencing to identify micro RNAs that are up-regulated in pluripotency, but down-regulated in differentiated cells. In addition, they should not be upregulated in mature cells and downregulating these micros in pluripotent stem cells should induce differentiation. These microRNAs would also be present in aggressive cancers. To identify micro RNAs that dominate differentiation, one would similarly look for up-regulation of micro RNAs that were not present in the pluripotent state but highly expressed in differentiating stem cells or highly expressed when moderators of pluripotency, such as MUC1*, are antagonized. These micros would also be downregulated in aggressive cancers.

To identify microRNAs that uniquely identify different subtypes of cancer, one would identify those micros that are present only in that cancer subtype, absent in healthy mature cells, and when down-regulated inhibit the growth of that specific subtype of cancer. MicroRNAs that identify subtypes of cancer may also be present in proliferating stem or progenitor cells. Several techniques are available for identifying microRNAs such as whole transcriptome analysis, Deep Sequencing, ChIP assays as well as computational methods and programs that predict the sequence of a microRNA that would suppress targeted genes. One way to classify cancers is whether they are MUC1*-positive or negative. Genes and gene products that uniquely identify undifferentiated cells, differentiated cells and cancer subtypes are similarly identified. In addition to microRNAs being used as a treatment for cancer or its prevention, genes or the proteins that are expressed when the unique identifier microRNAs are expressed can be administered rather than the microRNAs. Small molecules that upregulated targeted microRNAs, genes or gene products could alternatively be used as the therapeutic agents.

Combinations of elements that deliver a regulatory cluster signal, such as nucleic acids, micro RNAs, or proteins, that are dominant in newly differentiating stem cells can be administered as a new kind of cancer vaccine. Combinations of these elements that are dominant in different subtypes of cancer can be administered as a new type of cancer vaccine.

MUC1-negative cancer cells that have been transfected with MUC1* can also be administered as a cancer vaccine because they deliver mixed fundamental signals.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. The following examples are offered by way of illustration of the present invention, and not by way of limitation.

### EXAMPLES

Example 1. Inhibition of MUC1* or NM23 induces expression of miR-145 and triggers differentiation.

Human embryonic stem cells, H9s, were cultured on Matrigel for three passages by either: a) the standard method of 4ng/ml bFGF and 50% conditioned media from mouse fibroblast feeder cells (MEF); or b) 8nM NM23-S120G in minimal stem cell media; or c) 80ng/ml rabbit polyclonal anti-MUC1* antibody (raised against the full PSMGFR sequence). Minimal Stem Cell Media ("MM") 500 mL formula is: 400 ml DME/F12/GlutaMAX I (Invitrogen #10565-018), 100 ml Knockout Serum Replacement (Invitrogen# 10828-028), 5ml 100x MEM Non-essential Amino Acid Solution (Invitrogen# 11140-050), 0.9 ml (0.1mM) beta-mercaptoethanol (55mM stock, Invitrogen# 21985-023 - optional), 2.5 ml PSA (penicillin, streptomycin, amphotericin) MP Biochem (#1674049 optional).

The pluripotent stem cells grown using the three methods were then allowed to differentiate by withholding bFGF, NM23-S120G, or the anit-mUC1* antibody, respectively. In addition to withholding the NM23-S120G, in another aliquot, a synthetic PSMGFR peptide was added to cells grown according to (b) to bind to NM23 and prevent its interaction with MUC1*. Triplicates of each conditions were cultured so that RNA could be extracted at 3 different time points: 48, 96 and 144 hours. The cells were pelleted and frozen. Total RNA was extracted from the samples using the mirVana™ kit (Applied Biosystems, P/N: AM1561) per manufacturer's instructions. For each total RNA sample, two cDNA samples were synthesized using the TaqMan® MicroRNA Reverse Transcription Kit (Applied Biosystems, P/N: 4366596) and two different stem-loop primers specific for miR-145 and the small nuclear RNA U6B (RNU6B), which served as an endogenous control. Quantification of miR-145 and RNU6B in the cDNA samples was performed using TaqMan® MicroRNA Assays (Applied Biosystems, P/N: 4427975) per manufacturer's instructions. The real-time PCR data were analyzed using the comparative Ct method. The relative amount of miR-145 in each sample was obtained by computing the difference between the miR-145 Ct and the corresponding RNU6B Ct (ΔCt). A second normalization was performed by subtracting the smallest ΔCt from all the others in the data set (ΔΔCt).

The amount of miR-145 expression at each of the time points, compared to a standard was plotted and is shown in Figure 1. Recall that the microRNA miR-145 is the dominant microRNA that regulates differentiation from pluripotency to differentiation. The graph shows that the addition of the PSMGFR peptide that binds to and blocks the action of NM23, causes a spike in the expression of miR-145 that is a greater level of expression than the state of the art and peaks at 96 hours rather than the 144 hours that is typical of the state of the art method. This early and enhanced expression of miR-145 accelerates differentiation of stem cells and synchronizes their differentiation, which is important for directing differentiation to obtain a population of functional cells. It is also noted that withholding NM23 or anti-MUC1* resulted in an earlier rise in miR-145 expression than the control, state of the art FGF growth method.

### Example 2. Expression and characterization of NM23 wild type and variants that have distinct multimerization properties

### NM23-S120G cloning

WT NM23-H1 cDNA was amplified by polymerase chain reaction (PCR) using the following primer: 5'-atc gat gga tcc gat ggc caa ctg tga gcg tac c-3' (SEQ ID NO:2) and 5'- gtg gtg ctc gag ttc ata gat cca gtt ctg agc-3' (SEQ ID NO:3) (Integrated DNA technologies). After digestion with BamHI and XhoI restriction enzymes (New England Biolabs), the purified fragment was cloned into the pET21b vector (Novagen) digested with the same restriction enzymes. The pET21b vector carries an N-terminal T7-Tag sequence, a C-terminal HiTag® sequence and cloned target expression, driven by a T7 promoter. After sequence confirmation, we generated the NM23H1 mutant S120G (serine #120 mutated to a glycine) using the GeneTailor™ Site-directed mutagenesis system (Invitrogen) following the manufacturer instructions. Mutagenesis reaction was performed with 31.25 ng of methylated DNA template using the following primers: 5'-gcaggaacattatacatggcggtgattctg-3' (SEQ ID NO:4) and 5'-gccatgtataatgttcctgccaacttgtat-3' (SEQ ID NO:5). The PCR reaction consisted of a 2 min denaturation step at 95°c followed by a three step cycling (x35): denaturation for 15s at 95°c, annealing for 30s at 58°c and extension for 10min at 68°c. The amplified fragment was then cloned into DH5α ™-T1^{R} cells (Invitrogen) and several cloned were selected for plasmid extraction and sequence analysis. The positive clone, containing the mutation was finally transformed into BL21 (DE3) cells (Invitrogen) for recombinant protein expression.

### NM23-WT (wild type) cloning

WT NM23-H1 cDNA was amplified by polymerase chain reaction (PCR) using the following primer: 5'-atc gat gga tcc gat ggc caa ctg tga gcg tac c-3' (SEQ ID NO:2) and 5'- gtg gtg ctc gag ttc ata gat cca gtt ctg agc-3' (SEQ ID NO:3) ( (Integrated DNA technologies). After digestion with BamHI and XhoI restriction enzymes (New England Biolabs), the purified fragment was cloned into the pET21b vector (Novagen) digested with the same restriction enzymes. The pET21b vector carry an N-terminal T7-Tag sequence, a C-terminal HiTag® sequence and cloned target expression I driven by a T7 promoter. Several clones were selected for plasmid extraction and sequence analysis. The positive clone, was transformed into BL21 (DE3) cells (Invitrogen) for recombinant protein expression. The wild type protein was expressed and the secreted protein was collected and affinity purified on a His Gravitrap™ 1mL column (GE healthcare).

### NM23-S120G and NM23-WT expression/purification

500 mL LB broth (Luria-Bertani broth) was inoculated with one colony and cultured over night at 37°c. The next day 1L of LB was inoculated with 100 mL of overnight culture and cultured at 37°c until OD600 reached 0.5. At this point, recombinant protein expression was induced with 0.4mM Isopropyl-β-D-thio-galactoside (IPTG, Sigma) and culture was stopped after 4h.

### Purification Protocol #1: Secreted protein collected and purified

After harvesting the cells by centrifugation (6000 rpm for 10 min at 4°c), cell pellet (from 350 mL culture) was resupended into 30 mL of BugButer™ mater mix (Novagen) containing 100 mM phenylmethylsulfonyl fluoride (PMSF) and 100 mM Benzamidine. Cell suspension was incubated on a rotating platform (275 rpm) for 10 min at 25°c and insoluble cell debris was removed by centrifugation (20000rpm for 30 min at 4°c). The cleared lysate was then supplemented with 40mM imidazole and loaded onto a His Gravitrap™ 1mL column (GE healthcare) equilibrated with the following running buffer: phosphate buffer saline (PBS) pH 7.4, 40mM imidazole and 0.05% tween 20. The column was then washed with running buffer until the absorbance at 280nm was less than 0.01. The protein was then eluted off the column with PBS pH7.4 containing 500mM imidazole into 2mL fractions. The fractions containing the protein were combined, diluted to 0.1mg/mL in PBS pH7.4 and dialysed against PBS pH7.4. The protein was then aliquoted and stored at - 80°c.

This protocol produced variable results. The protein from each batch needed to be characterized by FPLC and native gel to determine which of the two typical outcomes had occurred: 1) about 50% dimer plus some tetramer and hexamer; or 2) 100% hexamer.

### Purification Protocol #2: Refolding and purified protocol produces 100% dimer NM23-S120G

After harvesting the cells by centrifugation (6000 rpm for 10 min at 4°c), cell pellet (from 350 mL culture) was resupended with running buffer: PBS pH7.4, 360 mM NaCl and 80 mM imidazole. Then lysozyme (1 mg/mL, Sigma), MgCl₂ (5mM) and DNAse (.5ug/ml) was added. Cell suspension was incubated on a rotating platform (275 rpm) for 30 min at 37°c and sonicated on ice for 5 min (power at 30%, 5sec on and 10 sec off). Insoluble cell debris was removed by centrifugation (20000rpm for 30 min at 4°c). The cleared lysate was then applied to a NiNTA column (5 mL, Qiagen) equilibrated with the running buffer. The column was washed with 6CV (column volume) of running buffer before eluting the protein off the column with 8CV of the running buffer supplemented with 420 mM imidazole (5 mL fractions). The elution fractions were analyzed on a non-reducing SDS-PAGE and fractions containing the protein were combined and concentrated to 7.5mL. Then 7.5 mL of denaturing buffer (100mM Tris pH 8.0 and 8M urea) was added to the protein and the resulting fraction was concentrated by half. This was repeated twice to reach a final urea concentration of 7M. The denatured protein was then subjected to refolding by dialysis. The protein was dialyzed successively for 24h against: 1) 100mM Tris pH8.0, 4M urea, 250 mM imidazole, 0.4M L-Arginine, 1mM EDTA and 5% glycerol, 2) 100mM Tris pH8.0, 2M urea, 100 mM imidazole, 0.4M L-Arginine, 1mM EDTA and 5% glycerol and 3) 100mM Tris pH8.0, 1M urea, 100 mM imidazole, 0.4M L-Arginine, 1mM EDTA and 5% glycerol. The protein was then dialysed against 100mM Tris pH8.0, 100 mM imidazole, 0.4M L-Arginine, 1mM EDTA and 5% glycerol for 9h and against 25mM Tris pH8.0, 100 mM imidazole, 0.1M L-Arginine, 1mM EDTA and 5% glycerol overnight. Finally, the protein was dialyzed against PBS pH7.4, 100mM imidazole, 1mM EDTA and 5% glycerol for 24h with one buffer change.

The refolded protein was analyzed on non-reducing SDS-PAGE and we observed the presence of dimer (~37KDa) but also the presence of higher molecular weight oligomers. The dimer was further purified by size exclusion chromatography using a Superdex 200 10/300 GL column (GE healthcare) on an Akta purifier 10 (GE healthcare) using PBS pH7.4 as running buffer. Such purification step was very efficient in isolating the refolded NM23-H1 S120G dimer with purity approaching 100% dimer form.

### FPLC Characterization of NM23-wild type and S120G mutant

Aliquots of several different preps of NM23-S120G or NM23-WT were tested to determine the percentage of each multimerization state, especially dimers, because this was the form that activates the MUC1* growth factor receptor pathway via dimerization of its extra cellular domain.

500 ul of each sample was loaded onto a Superdex 200 10/300 GL FPLC instrument. NM23-WT was at 0.17 mg/ml, NM23-S120G (hexamer batch) was loaded at 0.19 mg/ml, and NM23-S120G (dimer batch) was loaded at 0.15 mg/ml.

Figure 2 shows the overlay of the FPLC traces. NM23-WT has a major peak at 95 KDa, indicating it is comprised essentially of hexamer; a batch of NM23-S120G collected from secreted protein also has a its major peak at 95 KDa corresponding to hexamers; another batch of NM23-S120G shows it is comprised of about 50% dimer with major peaks at 30 KDa, minor peaks at 66KDa (tetramer) and 95KDa (hexamer); and a denatured and refolded NM23-S120G (protocol #2) produced a major peak at 30KDa (dimer) and minor peak at 15KDa (monomer). The recombinant protein has a calculated molecular weight of 19.1KDa, including weight of an affinity tag.

Non-denaturing and non-reducing gel analysis shows multimerization state of NM23-WT (wild type) compared to different preparations of NM23-S120G.

Various samples of NM23-WT and NM23-S120G preparations (secreted versus refolded) were run on a non-denaturing gels or non-reducing gels to determine the multimerization state of various expression or re-folding methods. On a non-denaturing gel, the molecular weights of the various NM23 multimers can be directly visualized. On a non-reducing gel, the dimer is kept intact by disulfide binding and runs with an apparent molecular weight of about 37KDa, while the hexamer runs with an apparent molecular weight of about 200KDa (monomer due to loss of disulfide bonds). The native gel shown in Figure 3A shows that NM23-WT is comprised mostly of hexamer, one batch of secreted NM23-S120G is also essentially 100% hexamer, while a different batch shows about 50% dimer with some tetramer and monomer. A denatured then refolded protocol produced NM23-S120G that was 70-80% dimer with some tetramer, hexamer and monomer present as well The denatured and re-folded NM23-S120G prep that results in about 70-80% dimer, was then purified by gel filtration to be 100% dimer, see Figure 3B. Note that the concentrations at which the samples were loaded 4mg/ml-0.1mg/ml are more than a thousand times more concentrated than the physiologically relevant concentrations at which NM23 exists in a cell, in the conditioned media from fibroblast cells, or that promotes undifferentiated stem cell growth. Therefore, the hexamer prep and wild type prep may equilibrate at the lower concentrations such that they also comprise more dimers than the gel reveals. The two SPR experiments described in the examples below support this conclusion.

Surface Plasmon Resonance (SPR) measurement of various NM23 multimers binding to a MUC1* extra cellular domain peptide (PSMGFR) that was immobilized on the chip.

Biacore chips were stripped of their surface and coated with self-assembled monolayers (SAMs) according to methods of Bamdad, C. The use of variable density self-assembled monolayers to probe the structure of a target molecule. Biophys J. 1998 Oct;75(4):1989-96. NTA-Ni-tri-ethylene glycol SAMs were formed to present 3.8% NTA-Ni. Histidine tagged PSMGFR peptide was flowed over the chip surface and immobilized to saturation. Next, each of the different preparations of NM23-wt or NM23-S120G (60% dimer) were injected over a stable peptide surface. Either NM23-WT or NM23-S120G was flowed over the peptide surface at five concentrations: 4, 8, 16, 32, and 64nM. The resultant binding curves shown in Figure 4 show that the amount of binding increases linearly as the concentration of NM23 increases linearly. However, the wild type (WT) protein only shows 150RUs of binding at the highest concentration (Figure 4A), while the 60% dimer prep shows nearly 400RUs (Figure 4B). These results indicate that the dimer form of NM23 preferentially binds to the MUC1* extra cellular domain peptide. Further analysis indicates that NM23 hexamer does not bind the MUC1* peptide at all and that the 100RUs of binding represent the small fraction of the preparation that existed as a dimer. Consistent with this interpretation, kinetic analysis showed very similar on and off rates for the species that bound from the two different preparations.

Other NM23-S120G preparations that showed different percentages of dimer present were also analyzed for binding to an SPR chip coated with a SAM comprised of either 3.8% (Figure 5A) or 5.7% (Figure 5B) NTA in a background of tri-ethylene glycol terminated C₁₁ thiols, to which was immobilized a layer of histidine-tagged PSMGFR peptide. Figure 5 shows that the preparation that produced 100% hexamer bound very little (70RUs) compared to the 60% dimer (408RUs) and the 70% dimer (547RUs) preparations. These results further demonstrate that the amount of binding to the layer of MUC1* extra cellular domain peptide (PSMGFR) was purely a function of dimer concentration. Inserts show non-reducing gels that quantified the amount of dimer present in each NM23 prep (Figure 5C).

In control experiments, an irrelevant peptide was immobilized on the same chip and a minimal amount of background binding resulted, which was subtracted from the measurement shown.

### Example 3. The effect of different NM23 multimerization states on the differentiation state of human embryonic stem cells: dimers promote pluripotent growth, hexamers whether from mutant NM23 or wild type, induce differentiation.

The effect of the multimerization state of NM23 on growing pluripotent stem cells was tested. Recombinant NM23-S120G can be expressed, refolded or purified to yield populations that are: 1) 100% dimers; 2) 100% hexamers; or 3) 50% dimers. Recall that direct binding data indicated that the hexamer form does not bind to a synthetic peptide with sequence of the extra cellular domain of MUC1* (Example 2). In addition, to the three preparations of the S120G mutant, NM23-WT (wild type) was prepared and characterized by FPLC and native gel. NM23-WT was shown to be comprised of mostly of hexamer. However, recall that these analyses were done at concentrations higher than what is secreted by stem cells and we therefore expect the wild type to be comprised of monomer, dimer, tetramer and hexamer until as its concentration increases, the population is rapidly taken over by the hexamer form.

The four preparations of NM23 were separately added to stem cell minimal media and used to culture Human H9 embryonic stem cells that had been plated onto Vita (Thermo Fisher, Waltham, MA) cell culture plates that had been coated with 12.5 ug anti-MUC1* extra cellular domain antibody. Additionally, a synthetic peptide having sequence of the MUC1* extra cellular domain peptide (PSMGFR) was added to cells receiving the NM23-S120G 100% dimer preparation in order to block interaction between NM23 and MUC1* receptor by binding to NM23. Similarly, an Fab of an anti-MUC1* antibody was added to another aliquot of cells cultured in the NM23-S120G 100% dimer preparation to competitively inhibit interaction between NM23 and MUC1* by binding to the MUC1* receptor.

Images of the stem cells taken on Day 3 are shown in Figure 6. The results were that after 3 days, the NM23-S120G 100% dimer prep caused the stem cells to grow the fastest and completely undifferentiated (Fig. 6A). When the interaction was disrupted by either the PSMGFR peptide (Fig. 6B) or the anti-MUC1* Fab (Fig. 6C), the stem cells very quickly differentiated; the effect of blocking NM23 or blocking the MUC1* extra cellular domain was equivalent and no differences between these batches of stem cells could be detected. Nearly as differentiated were cells grown in the 100% hexamer prep of NM23-S120G (Fig. 6D), which was indistinguishable from the effect of growth in NM23-WT (wild type) (Fig. 6E). The stem cells cultured in the NM23-S120G prep that was 50% dimer had some differentiation (Fig. 6F), but not as extensively differentiated as caused by the hexamer of wild type NM23.

These results confirm that dimeric NM23 is the form that activates the MUC1* receptor to promote pluripotent growth and inhibits differentiation. Results further show that interrupting this interaction initiates differentiation. Even further, the results show that wild type NM23 is involved in a self-regulating feedback loop wherein as NM23 concentration increases, for example via increasing numbers of stem cells all secreting the protein, it forms hexamers that do not bind to the MUC1* receptor, which in turn triggers differentiation. Mutants, variants, chimeras of NM23 proteins, or even refolding protocols that generate stable dimers are valuable for stem cell growth and for delivering a pluripotency signal. Alternatively, mutants, variants, chimeras of NM23 proteins, or even refolding protocols that generate stable hexamers are valuable for delivering signals that induce differentiation and thus are useful for the treatment or prevention of cancers.

### Example 4. NM23 and MUC1* co-localize at cell surface then translocate to the nucleus.

Immunofluorescence experiments were performed to identify the subcellular trafficking and localization of MUC1* and/or its ligand NM23 to the nucleus. Human breast cancer cells, T47D, a MUC1*-positive cell line, were obtained from the American Type Culture Collection (ATCC) and maintained in RPMI media containing 10% FBS. 8-well chamber glass slides (Lab-Tek, #154534) were pre-coated with type 1 collagen solution (Sigma, #C8919) to facilitate cell adherence. T47D cells at 1.25 x 10⁵ cells/ml were plated on precoated Lab-Tek 8-well chamber slides and serum starved for 24 -48 hours in the presence of RPMI containing 1% FBS.

NM23-S120G (50% dimer prep, see Examples 2 and 3) or NM23-WT (wildtype) were added at varying concentrations (8, 16, 32, 64, 128 nM) for 30, 60 and 120 min. Cells were then fixed in 4% paraformaldehyde solution for 10 min at room temperature. Following fixation, cells were washed three times, for 5 min/wash in phosphate buffered solution (PBS). Block non-specific staining and cell permeabilization by incubation for one hour at room temperature in blocking buffer containing PBS with 0.1% bovine serum albumin (BSA), 0.05% saponin and 5% normal goat serum. Primary antibodies to the extra cellular domain of MUC1* (C3 mab, Minerva Biotechnologies: raised against GTINVHDVETQFNQYKTEAASRYNLTISDVSVSDV (SEQ ID NO:6) and NM23 (C20, Santa Cruz Antibodies, CA) were added in the presence of blocking buffer for one hour at room temperature. Cells were washed three times, for 5 min/wash in phosphate buffered solution (PBS) containing 1% BSA and 0.05% saponin. Secondary antibodies (Alexa-fluor 488 and Alexa-fluor 555, Invitrogen) were applied in blocking buffer at a final dilution of 1:100 and incubated for one hour at room temperature in the dark. Cells were then washed twice in PBS for 5 min/wash followed by one wash in water for 2 min. Cells were then mounted in Prolong Gold containing the nuclei stain DAPI (Invitrogen) and cover glass (size 1.5) was applied.

For confocal imaging, a Zeiss LSM 510 confocal microscope was used (Harvard Digestive Disease Confocal Core Facility, Beth Israel Deaconess Medical Center, Boston, MA). Lab-Tek 8 well chamber slides were placed on the stage of a Zeiss LSM 510 confocal microscope. Representative cells were focused using either a Plan Neofluar x40/1.3 oil immersion lens or a Plan Neofluar x20/0.50 lens. Alexa-488 labeling was excited using a 488 nm laser line of a 25 mW Argon excitation source, Alexa-555 labeling was excited using a 543 nm laser line of a 1mW HeNe laser and DAPI labeling was excited using a 405 nm laser line. Eight-bit images were collected and the pinhole was set to 1 Airy unit or 76 µm for all three wavelengths and images were collected at 0.11 µm/pixel resolution The gain and offset were set to expand the signal from the cell being imaged over the 255 gray value scale of the 8-bit data range. Z-stacks (0.5 - 1.0 µm intervals from cell bottom to top) were acquired every 1 - 2 min for a total of 5 - 7 minutes.

Results are shown in Figure 7. Figure 7 (top row) shows that NM23-WT (wild type), which is mostly hexamer, does not exhibit increasing co-localization with MUC1* or translocation to the nucleus with increasing concentration of NM23-WT supplied. Conversely, NM23-S120G (dimer) localized to the nucleus in a concentration-dependent manner following a 30 min incubation period. Nuclear localization was greatest at 8nM as indicated by the white arrows.

### NM23 is a ligand of MUC1*

The NM23 mutant, S120G exists predominantly as a dimer and binding to MUC1*, dimerizes the receptor. To show that the MUC1*-NM23 complex is localized to the nucleus we performed another experiment, carried out as described above. T47D cells were incubated for 60 min in the presence of varying concentrations of S120G-NM23. Figure 8 shows MUC1* and NM23 co-localized, as indicated by the yellow color, following incubation with S120G-NM23 with both cytoplasmic, then nuclear localization. These results are consistent with the idea that the MUC1*-NM23 receptor complex localizes to the nucleus and regulates transcription. Note the concentrations of NM23-S120G that are being tested. We have observed that 16nM NM23-S120G is ideal for stimulating human stem cell growth and for inhibiting differentiation. We have also observed that at 64nM and above, stem cell growth is inhibited and differentiation progresses. These results are consistent with the fact that MUC1* receptor is a Class I growth factor receptor that is activated by dimerization. At concentrations of 64nM and above, one NM23 dimer is bound to each MUC1* receptor rather than one NM23 dimer binding to two MUC1* receptors.

Because we know that the NM23-MUC1* interaction promotes pluripotent stem cell growth as well as cancer cell growth, it is concluded that the NM23 and MUC1* regulate the transcription of factor that promote pluripotency and cancerous growth.

### Example 5. NM23 and MUC1* translocation to the nucleus results in genes that promote pluripotency being up-regulated and genes that induce differentiation being down-regulated.

Following MUC1* stimulation with NM23, especially preparations or variants that prefer dimerization, analyses are performed to reveal the identity of the genes, gene products, microRNAs and other regulatory nucleic acids that are up- or down-regulated in response to NM23 and/or MUC1* translocation to the nucleus. Antibodies raised against NM23 and against the extra cellular domain of MUC1* such as anti-PSMGFR antibodies or antibodies directed against the MUC1 cytoplasmic tail are added to nuclear preps and pull-down assays are carried out such that all nucleic acids and all proteins associated with a MUC1* or an NM23 complex are immune-precipitated. The sequences of the nucleic acids are readily determined by sequencing and the identity of the proteins are determined by antibody probing, mass spec, MALDI mass spec and the like, known to those skilled in the art. ChIP assays, ChIP SEQ, PCR, RT-PCR as well as Deep Sequencing analysis are ideal for such analyses.

For example, chromatin immunoprecipitation (ChIP) assays are performed with anti-NM23, anti-MUC1* or anti-MUC1-CT (cytoplasmic tail) antibodies. Promoter sequences isolated using this technique are identified by amplification and labeling of immunoenriched DNA sequences, and hybridization to promoter array chips, or amplified and sequenced directly, using the SOLiD3 analyzer. Direct sequences will identify promoter binding sites of genes that are regulated directly or indirectly by MUC1. To determine whether genes regulated by NM23 and/or MUC1 are up- or down-regulated, the SOLiD3 analyzer can be used in conjunction with RT-PCR that will only amplify genes downstream of the promoter sites identified that are bound directly or indirectly by nuclear MUC1* or MUC1-CT. Quantitation of total mRNA and data analysis methods could be used as an alternative method.

Alternatively, total transcriptome analysis is done following stimulation with NM23 and compared to total transcriptome analysis that is done with cells that have not been stimulated with NM23.

In addition to comparing cells that have and have not been stimulated by NM23, comparing different cell types is also contemplated. For example, by comparing stimulated and unstimulated MUC1*-positive cancer cells to MUC1*-negative cancer cells, one can identify those genes, nucleic acids, microRNAs and proteins whose action is limited to function involving MUC1*-mediated growth. In another aspect, the stimulated cells are pluripotent stem cells which are then compared to both newly differentiating stem cells and to MUC1*-positive cancer cells. Preferred cells to be analyzed according to these methods are any MUC1*-positive cells.

### Example 6. Human stem cells cultured in NM23-S120G absent FGF or mouse feeder cells stain positive for markers of the Naïve state, which is the true human pluripotent stem cell state.

Human stem cells cultured in either NM23-S120 (50% dimer) or FGF over human or mouse feeder cells are probed for the presence of markers of either the Naive state (true pluripotent state) or the Primed state.

We started with H9 embryonic stem cells that were Primed. They had been cultured in bFGF and over mouse embryonic fibroblast (MEF) feeder cells for approximately 30 passages. A first set of cells continued to be cultured in bFGF over MEFs. A second group was transitioned onto human feeder cells (HS27s) but still cultured in 4ng/ml of bFGF. A third set of cells was cultured in NM23-S120G, but remained on the mouse MEF feeder cells. A fourth set of the cells was transitioned onto human feeder cells (HS27s) and cultured in NM23-S120G. All cells were cultured according to these conditions for an additional 6 passages. The cells were then stained for the presence of Klf4 which is a marker for Naive stem cell state and Foxa2 which is a marker for the Primed stem cell state. Figures 9 and 10 show that only cells cultured in NM23 and exposed to human feeder cells expressed Naive stem cell marker Klf4 and did not express any Foxa2 which is the Primed stem cell marker.

### Example 7. Human stem cells cultured in NM23-S120G and grown over human feeders or a MUC1* antibody surface function as Naïve stem cells.

Vita^{™} (Thermo Fisher, Waltham, MA) cell culture plates were coated with an anti-MUC1* antibody (12.5ug per well of a 6-well plate) and incubated overnight at 4 degrees C. Experiments showed that human stem cells cultured in FGF would not adhere to these surfaces unless first pre-incubated in NM23 and unless a Rho Kinase inhibitor (Y-27632) was added. However, if the source cells were cultured in NM23 over human feeder cells or over a defined surface such as a layer of anti-MUC1* antibodies, then the need for the Rho kinase inhibitor was eliminated, and they bound efficiently to the Vita-anti-MUC1* antibody surface. Figure 11 shows images of human H9 embryonic stem cells that had been cultured in NM23-S120G (8nM in minimal stem cell media) over HS27 human feeder cells, (left two columns; Figs. 11A, B, D, E, G, and H) or cultured in bFGF over mouse embryonic fibroblast (MEFs) feeders (right column; Figs. 11C, F and I). The top row (Figs. 11A, B, C) shows cells that were trypsin harvested then plated onto Vita-anti-MUC1* surfaces; middle row (Figs. 11D, E, F) shows cells harvested with EDTA treatment, and bottom row (Figs. 11G, H, I) shows cells harvested with trypsin but plated in the presence of a Rho kinase inhibitor (ROCi) (Y-27632). Cells pictured in the left column (Figs. 11A, D, G) and the right column (Figs. 11C, F, I) were in plates that were centrifuged at 1500RPMs for 5 minutes to bring cells to the surface. Cells in the middle column (Figs. 11B, E, H) were not centrifuged. Figure 11 shows that cells from an NM23, human feeder source did not benefit from Rho kinase inhibitor, but cells from an FGF-MEF source did not produce undifferentiated colonies unless treated with Rho kinase inhibitor and pre-incubated with NM23 (S120G 50-100% dimers) for 30 minutes prior to plating.

Additionally, human iPS cells that were grown in 8nM NM23-S120G (50% dimer formulation) attached to the Vita-MUC1* antibody surfaces well in the absence of Rho kinase inhibitor, see Figure 17. ES or iPS cells that had been grown on Vita-anti-MUC1* surfaces for 2 or more passages also did not require Rho kinase inhibitor. These results are consistent with: 1) the Vita-anti-MUC1* surfaces preferentially binds to Naive stem cells; 2) NM23 (dimers) grown stem cells over human feeders or the Vita-anti-MUC1* surface revert to the Naive state; and 3) Rho kinase inhibitors in addition to pre-incubation in NM23-S120G revert Primed stem cells to the Naive state. Thus, surfaces coated with a MUC1* antibody raised against the PSMGFR peptide or the peptide selectively bind human Naive stem cells and can be used to separate Naive stem cells from Primed stem cells. Especially preferred are Vita (ThermoFisher) plates coated with the monoclonal antibody C3 mab raised against the peptide GTINVHDVETQFNQYKTEAASRYNLTISDVSVSDV (SEQ ID NO:6).

Recall that in Example 6, we showed that human stem cells grown in this way (NM23 over human feeders) bore the markers of Naive stem cells. These results indicate that surfaces presenting anti-MUC1* antibodies, especially when attached to surfaces having chemical composition similar to the Vita plate, selectively bind Naive stem cells. It has been reported that human stem cells cultured in FGF over MEFs benefitted greatly from treatment with Rho kinase inhibitor: more colonies, better growth¹⁸. Mouse embryonic stem cells showed no benefit from growth in the presence of Rho kinase inhibitor, however, mouse stem cells from the epiblast did. Recall that Jaenisch et al reported that mouse embryonic stem cells were the equivalent of the elusive human *Naïve* stem cells, while mouse stem cells taken from the epiblast stage were equivalent to human *Primed* stem cells. Therefore, these results, in light of our results indicate that Primed stem cells can be coerced to become more Naive by treatment with Rho kinase inhibitor and/or NM23. See Figure 11.

In the present application, we show that human stem cells and particularly Naive stem cells preferentially bind to anti-MUC1* antibody coated surfaces. Vita^{™} surfaces of WO2009/105570, the contents of which are incorporated by reference herein for its disclosure of the Vita^{™} surface, have been used in some of these examples, but more generally the invention contemplates anti-MUC1* antibodies coated onto surfaces that are comprised of approximately 1.7-2.1% Nitrogen, 26.4-28.7% Oxygen and 28.2-30.7% Nitrogen and Oxygen combined, wherein the surface has a water contact angle of 14.3-18.8 degrees.

### Example 8. Cancer cells belonging to a first subtype cannot be grown in the conditioned media from cancer cells of a second subtype.

U87 cells, which are a MUC1*-positive glioma cell line, were cultured in the conditioned media from either a MUC1*-positive cancer cell line (breast cancer, T47D) or a MUC1*-negative cancer cell line (prostate cancer, LnCAP). We note that different media are recommended for growing different cell types. To ensure that differences seen were not due to different media that cells were grown in, all cells were grown in the same media which was an even 33/33/33 or 50/50 combination of the recommended media. Figure 12 shows that the U87 glioma cells proliferated well when cultured in the conditioned media of the MUC1*-positive cells but would not proliferate in the conditioned media of the MUC1*-negative cells. These results show that regulatory elements secreted by a first cell type interfere with the internal programming of a second cell type, wherein the mixed signal triggers a default mechanism that sends the cell into growth stasis.

### Example 9. Conditioned media from cells transfected with a foreign protein is comprised of a mixture of regulatory signatures which inhibit the growth of a second cell type.

In another experiment carried out as described above, U87 cells were grown in either the conditioned media of MUC1*-negative cancer cells (HCT-116 colon cancer, "vector") or that same cell line into which MUC1* had been transfected ("FLR"). The HCT-116 cells and the MUC1*-HCT-116 transfected cells were grown in a 50/50 mix of the media recommended for growth of U87s and minimal stem cell media. Figure 13 shows that the conditioned media from the MUC1*-negative cells did not allow growth of U87 cancer cells, and although the conditioned media of those cells transfected with MUC1* allowed some growth, it is inhibited compared to the control cells. This result is consistent with the idea that the MUC1*-transfected into a MUC1-negative cell constitutes a first mixed signal, which when coupled with a second signal, wherein signal means a signature of regulatory elements particular to a sub-type of cells, delivers a second mixed signal which inhibits cancer cell growth.

### Example 10. Conditioned media from human stem cells has a signature that reprograms cancer cells to limit their self-replication.

MUC1*-positive breast cancer cells, T47Ds, were grown in conditioned media from other MUC1*-positive cancer cells. The conditioned media of the other MUC1*-positive cancer cells did not have an adverse effect on the growth of the MUC1* positive cells, Figure 14A.

In a similar experiment, MUC1*-positive breast cancer cells, T47Ds, were cultured in the conditioned media from either undifferentiated human ES cells (H9s) or in the conditioned media of newly differentiating H9s. Figure 14B shows that the conditioned media from the differentiating stem cells immediately ceases growth of T47Ds. The conditioned media from the undifferentiated stem cells initially increased growth but then after 144 hours, inhibited their growth, presumably when the regulatory elements that the stem cells retain, but that cancer cells have lost, initiate programmed differentiation.

### Example 11. Conditioned media from MUC1*-positive cancer cells programs human stem cells so that they remain in the self-replicating, undifferentiated state; MUC1*-negative cancer cells deliver to stem cells a mixed regulatory signal and sends the stem cells into stasis.

In this experiment, undifferentiated human stem cells (BG01V/hOG (Invitrogen, Carlsbad, CA)) were grown in conditioned media from a panel of other cells to demonstrate that stem cells also could be sent into stasis by delivering to the cell, regulatory elements that belong to two different growth signatures.

Undifferentiated human stem cells as single cells (harder to grow) or as colony pieces were cultured in 8nM NM23-S120G (50% dimer) in either minimal stem cell media, supplemented with: a) nothing (control); b) conditioned media (CM) from MUC1* positive T47D breast cancer cells; c) conditioned media from HCT-116 cells (MUC1*-negative) that had been transfected with MUC1*; or d) conditioned media from HCT-116 cells (MUC1*-negative) that had been transfected with empty vector. The table in Figure 15 shows that the stem cells cultured in 50/50 minimal media and conditioned media from MUC1*-positive cancer cells proliferate well as undifferentiated pluripotent stem cells. Note that the stem cells that were plated as single cells grew better in MUC1* cancer cell conditioned media than in minimal media alone. However, there was a dramatic reduction in growth when the conditioned media from HCT-MUC1* transfectants was added. Moreover, the conditioned media from HCT-empty vector cells (MUC1*-negative) sent the cells into stasis and they did not proliferate. Images of the resultant cells, taken after 5 days in the indicated media, were taken and are shown in Figure 16.

In summary, pluripotent stem cells do not grow and cannot proliferate in the presence of conditioned media from MUC1*-negative cancer cells (HCT), but undergo enhanced proliferation when cultured in the presence of conditioned media from MUC1*-positive cells (T47D and HCT-MUC1*). These results are consistent with the idea that human pluripotent stem cells and MUC1*-positive cancer cells share a common proliferation program regulated by factors secreted by both these cell types. MUC1*-negative cancer cells transfected with MUC1* secrete factors that are inhibitory to stem cell growth, arguing that treating cells with a mixture of regulatory elements that are particular to different growth signatures, prevents cell growth and sends the cells into a stasis followed by cell death and therefore can be used as an anti-cancer treatment.

### Example 12. Categorizing cancer sub-types so that the signatures of their growth regulatory elements can be elucidated

A signature of growth regulatory elements means a subset of nucleic acids, microRNAs, genes or proteins that regulate the proliferation of a certain subset of cells, wherein the expression levels of these elements are significantly different from their levels in another cell type. For example, a signature of growth regulatory elements for a subset of breast cancers would be a collection of nucleic acids, microRNAs, genes or proteins that are significantly up- or down-regulated in comparison to a group comprised of several other cancer types. Cancer cells can be classified into sub-types whose proliferation is controlled by common regulatory elements such as microRNA clusters, by co-culturing the cells or culturing a first type of cancer cell in the conditioned media from another type of cancer cell. Cancer cells that proliferate without inhibition or stasis when cultured in the conditioned media of a second type of cancer cell, means that both cancer cell types fall into the same category, meaning their proliferation is controlled by a common signature of growth regulatory elements. When a first type of cancer cells cannot be cultured in the conditioned media of a second type of cancer cell, that means that they belong in different subtype groups and their growth is regulated by different microRNA clusters. The dominant microRNAs that control the growth of each subtype are determined using Deep Sequencing, RT-PCR, total transcriptome analysis, or the like. The dominant microRNA or microRNA cluster of one type can be used to treat or prevent the cancer of the other type.

All of the references cited herein are incorporated by reference in their entirety.

Those skilled in the art will recognize, or be able to ascertain using no more than routine experimentation, many equivalents to the specific embodiments of the invention specifically described herein. Such equivalents are intended to be encompassed in the scope of the claims.

### REFERENCES

1. Mahanta S, Fessler SP, Park J, Bamdad C. (2008) A minimal fragment of MUC1 mediates growth of cancer cells. PLoS ONE. Apr 30; 3(4):e2054
2. Hikita ST, Kosik KS, Clegg DO, Bamdad C. (2008) MUC1* mediates the growth of human pluripotent stem cells. PLoS ONE. Oct 3; 3(10):e3312
3. Fessler SP, Wotkowicz MT, Mahanta SK, Bamdad C. (2009) MUC1* is a determinant of trastuzumab (Herceptin) resistance in breast cancer cells. Breast Cancer Res Treat. May 5
4. Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. (2007) Induced pluripotent stem cell lines derived from human somatic cells. Science. Dec 21;318(5858):1917-20
5. Takahashi K, Tanabe K, Ohnuki M, Narita M, Ichisaka T, Tomoda K, Yamanaka S. (2007) Induction of pluripotent stem cells from adult human fibroblasts by defined factors. Cell. Nov 30; 131(5):861-72
6. Lyssiotis CA, Foreman RK, Staerk J, Garcia M, Mathur D, Markoulaki S, Hanna J, Lairson LL, Charette BD, Bouchez LC, Bollong M, Kunick C, Brinker A, Cho CY, Schultz PG, Jaenisch R. (2009) Reprogramming of murine fibroblasts to induced pluripotent stem cells with chemical complementation of Klf4. Proc Natl Acad Sci USA. 2009 May 15
7. Jacob Hanna, 1, Albert W. Chenga,b, Krishanu Sahaa, Jongpil Kima, Christopher J. Lengnera, Frank Soldnera, John P. Cassadya,c, Julien Muffata, Bryce W. Careya,c, and Rudolf Jaenisch, "Human embryonic stem cells with biological and epigenetic characteristics similar to those of mouse ESCs", PNAS, April 8, 2010.
8. Bader, Andreas G., Brown, David., and Winkler, Matthew. The Promise of MicroRNA Replacement Therapy. Cancer Res; 70(18), Sept 2010
9. Garzon, Ramiro, Marcucci, Guido, and Croce, Carlo M. Targeting microRNA's in cancer: rationale, strategies and challenges. Nature Reviews; 9, Oct 2010
10. Lu, Jun, Getz, Gad, Miska, Eric, A., et al. MicroRNA expression profiles classify human cancers. Nature; 435, June 2005
11. Neveu, Pierre, Kye, Min Jeong, Qu, Shuping, et al. MicroRNA Profiling Reveals Two Distinct p53-Related Human Pluripotent Stem Cell States. Cell Stem Cell; 7, Dec 2010
12. Martinez, Natalia J., and Gregory, Richard, I. MicroRNA Gene Regulatory Pathways in the Establishment and Maintenance of ESC Identity. Cell Stem Cell; 7, July 2010
13. Cao H, Yang C, Rana TM, 2008 Evolutionary Emergence of microRNAs in Human Embryonic Stem Cells. PLoS ONE 3(7): e2820
14. Boyer LA, Lee TI, Cole MF, Johnstone SE, Levine SS, Zucker JP, Guenther MG, Kumar RM, Murray HL, Jenner RG, Gifford DK, Melton DA, Jaenisch R, Young RA. (2005) Core transcriptional regulatory circuitry in human embryonic stem cells. Cell. Sep 23;122(6):947-56
15. Xu N, Papagiannakopoulos T, Pan G, Thomson JA, Kosik KS.(2009) A Repressive Role for MicroRNA-145 on OCT4, SOX2, KLF4 and Human Embryonic Stem Cell Pluripotency. Xu N, Papagiannakopoulos T, Pan G, Thomson JA, Kosik KS. Cell. May 15;137(4):647-58
16. Boyer LA, Lee TI, Cole MF, Johnstone SE, Levine SS, Zucker JP, Guenther MG, Kumar RM, Murray HL, Jenner RG, Gifford DK, Melton DA, Jaenisch R, Young RA. (2005) Core transcriptional regulatory circuitry in human embryonic stem cells. Cell. Sep 23;122(6):947-56
17. Lascu I, Giartosio A, Ransac S, Erent M (2000) Quaternary Structure of Nucleoside Diphosphate Kinases. J Bioenerg Biomemb 32: 227-236
18. Watanabe K, Ueno M, Kamiya D, Nishiyama A, Matsumura M, Wataya T, Takahashi JB, Nishikawa S, Nishikawa S, Muguruma K, Sasai Y, "A ROCK inhibitor permits survival of dissociated human embryonic stem cells", Nat Biotechnol. 2007 Jun;25(6):681-6

### Clauses:

The following clauses are not claims and represent different embodiments of the invention:
[1.] A method of treating a patient suffering from cancer, comprising administering to the patient a composition that contains (i) at least two regulatory elements, or (ii) an agent that causes expression of at least two regulatory elements unique to at least one cancer sub-type signature, wherein the cancer sub-type signature is different from the cancer subtype signature of the cancer that afflicts the patient.
[2.] The method according to clause 1, wherein the regulatory element is RNA, microRNA, or protein.
[3.] The method according to clause 1, wherein the agent that causes expression of at least two regulatory elements unique to at least one cancer sub-type signature is a small molecule.
[4.] The method according to clause 2, wherein the microRNA is microRNA-145.
[5.] The method according to clause 1, wherein the cancer the patient is afflicted with is a MUC1*-positive cancer and the regulatory elements unique to the at least one cancer sub-type signature are from MUC1*-negative cancer cells.
[6.] The method according to clause 1, wherein the cancer cells of the cancer subtype of the regulatory element cannot be co-cultured with the patient's cancer cells.
[7.] A method of treating a patient suffering from cancer, comprising administering to the patient a composition that contains (i) at least two regulatory elements, or (ii) an agent that causes expression of at least two regulatory elements unique to at least one stem cell sub-type signature.
[8.] The method according to clause 7, wherein the stem cell sub-type signature is that of a newly differentiating human stem cell or a differentiated cell.
[9.] The method according to clause 8, wherein the stem cell is Naive stem cell.
[10.] The method according to clause 7, wherein the cancer the patient is afflicted with is a MUC1*-positive cancer and the elements unique to the at least one stem cell sub-type signature are from a newly differentiating human stem cell or a differentiated cell.
[11.] The method according to clause 10, wherein the stem cell is Naive stem cell.
[12.] The method according to clause 7, wherein the regulatory element is RNA, microRNA, or protein.
[13.] The method according to clause 12, wherein the microRNA is microRNA-145.
[14.] The method according to clause 7, wherein the agent that causes expression of at least two regulatory elements unique to at least one stem cell sub-type signature is a small molecule.
[15.] A method of treating a patient suffering from cancer, comprising administering to the patient a composition that contains (i) at least two regulatory elements, or (ii) an agent that causes expression of at least two regulatory elements unique to at least one proliferation plateau signature.
[16.] The method according to clause 15, wherein the regulatory element is RNA, microRNA, or protein.
[17.] The method according to clause 16, wherein the microRNA is microRNA-145.
[18.] The method according to clause 15, wherein the agent that causes expression of at least two regulatory elements unique to at least one proliferation plateau signature is a small molecule.
[19.] A method for identifying regulatory element useful to treating cancer in person suffering from cancer comprising:
   (i) obtaining a first molecular composition of regulatory elements from human Naive pluripotent stem cells;
   (ii) obtaining a second molecular composition of regulatory elements from cells that have differentiated from the Naive pluripotent stem cells of step (i);
   (iii) comparing molecular composition of regulatory elements between the first and second molecular composition of regulatory elements; and
   (iv) identifying the regulatory element whose expression is significantly increased in the second molecular composition of regulatory elements.
   (v) obtaining a third molecular composition of regulatory elements from the cancer sub-type the patient is afflicted with;
   (vi) comparing the regulatory elements identified in (iv) with the third molecular composition of regulatory elements;
   (vii) identifying regulatory element that is absent or significantly reduced in the patient's cancer sub-type.
[20.] The method according to clause 19, wherein the regulatory element is RNA, microRNA or protein.
[21.] A method for identifying regulatory element useful to treating cancer in person suffering from cancer comprising:
   (i) obtaining a first molecular composition of regulatory elements from cancer cells;
   (ii) obtaining a second molecular composition of regulatory elements from human newly differentiating stem cells;
   (iii) comparing molecular composition of regulatory elements between the first and second molecular composition elements; and
   (iv) identifying the regulatory element whose expression is increased in the second molecular composition of regulatory elements.
[22.] The method according to clause 21, wherein the first molecular composition is from the patient's cancer sub-type.
[23.] The method according to clause 21, wherein the regulatory element is RNA, microRNA or protein.
[24.] A method of treating a patient suffering from cancer comprising administering to the patient a regulatory element that induces the growth of a different sub-type of cancer.
[25.] The method according to clause 24, wherein the regulatory element is absent from or is expressed at lower levels in the cancer afflicting the patient compared with the different sub-type of cancer.
[26.] The method according to clause 24, wherein the regulatory element is microRNA.
[27.] The method according to clause 26, wherein the regulatory element is microRNA-145.
[28.] A method for treating a patient with cancer or at risk of developing cancer comprising administering to the patient regulatory elements that are from at least two different and distinct cancer sub-type signatures.
[29.] The method according to clause 28, wherein the regulatory elements are chosen from microRNAs, nucleic acids, proteins or small molecules that mimic the activity of the regulatory elements.
[30.] A method of treating a patient with cancer or at risk of developing cancer, comprising administering to the patient at least two separate microRNAs, wherein one induces differentiation and the other induces pluripotency.
[31.] A method of treating a patient with cancer or at risk of developing cancer, comprising administering to the patient at least two different microRNAs, or microRNA clusters, wherein each microRNA or microRNA cluster is dominantly expressed in cancers of distinct subtypes.
[32.] The method according to clause 31, wherein the distinct subtypes of cancer are MUC1*-positive and MUC1*-negative.
[33.] A method of treating a patient with cancer, comprising administering to the patient microRNA, or microRNA clusters having characteristics of a subtype of cancer that is different from the patient's subtype of cancer.
[34.] A method for vaccinating a person against cancer by administering to a subject in need thereof a mixture of regulatory elements of at least two cancer sub-type signatures, wherein each uniquely identifies a different subtype of cancer.
[35.] The method according to clause 34, wherein the regulatory elements are microRNAs.
[36.] A method for propagating pluripotent stem cells without the need for Rho kinase inhibitor, comprising: culturing the cells with an agent that activates MUC1* growth factor receptor pathway.
[37.] The method according to clause 36, wherein the agent is NM23 and primarily exists in dimer form.
[38.] The method according to clause 37, further comprising growing the pluripotent stem cells over human fibroblast feeder layer.
[39.] The method according to clause 36, further comprising growing the pluripotent stem cells on a layer free of mouse embryonic fibroblast feeder cell components.
[40.] The method according to clause 36, further comprising culturing the cells on a MUC1* antibody surface.
[41.] The method according to clause 40, wherein the surface is Vita^{™} cell culture plate.
[42.] A method for propagating or maintaining Naive stem cells, comprising stimulating MUC1* pathway in the absence of mouse feeder cells or their extracts.
[43.] The method according to clause 42, further comprising culturing the cells on a MUC1* antibody surface.
[44.] The method according to clause 43, wherein the surface is Vita^{™} cell culture plate.
[45.] A method for separating Naive stem cells from a mixed population of cells, comprising contacting the mixed population of cells to a surface coated with MUC1* antibody, wherein the Naive stem cells bind to the MUC1* antibody surface.
[46.] The method according to clause 45, wherein the MUC1* antibody surface is formed on a Vita^{™} plate.
[47.] A method of categorizing types of cancers comprising determining which cancer cell types can grow in the conditioned media of another cancer cell type, wherein the ability to grow in the conditioned media of another cancer cell type means that the two cancers belong to the same sub-type.
[48.] A method of identifying regulatory elements that control the growth and differentiation of a cancer subtype comprising: first categorizing types of cancers according to clause 47, then performing molecular analysis including microRNA analysis and total transcriptome analysis to identify regulatory elements.
[49.] A method of determining suitability of a particular regulatory element treatment for a sub-type of cancer in a patient comprising employing the method according to clause 48 to identify the treatment that is effective for that cancer sub-type using the obtained regulatory element information.

## Claims

1. A method of categorizing types of cancers comprising determining which cancer cell types can grow in the conditioned media of another cancer cell type, wherein the ability to grow in the conditioned media of another cancer cell type means that the two cancers belong to the same sub-type.

2. A method of identifying regulatory elements that control the growth and differentiation of a cancer subtype comprising: first categorizing types of cancers according to claim 1, then performing molecular analysis including microRNA analysis and total transcriptome analysis to identify regulatory elements.

3. A method of determining suitability of a particular regulatory element treatment for a sub-type of cancer in a patient comprising employing the method according to claim 2 to identify the treatment that is effective for that cancer sub-type using the obtained regulatory element information.
